# EUROPÄISCHE PATENTANMELDUNG

(11) **EP 3 845 504 A1**
(43) Veröffentlichungstag der Anmeldung: **07.07.2021**
(21) Anmeldenummer: 19220016.0
(22) Anmeldetag: 30.12.2019
(51) Int. Cl.: C03C 3/097, C03C 3/11, C03C 4/00, C03C 4/02, C03C 10/12, C03C 10/16, C03C 23/00, C03C 4/04

(54) **VERFAHREN ZUR HERSTELLUNG EINER MEHRFARBIGEN DENTALRESTAURATION**

(71) Anmelder: Ivoclar Vivadent AG, 9494 Schaan (LI)
(72) Erfinder: Ritzberger, Christian, 9472 Grabs (CH); Dittmer, Marc, 6800 Feldkirch (AT); Rampf, Markus, 7212 Seewis (CH); Niedrig, Christian, 9464 Rüthi (CH)
(74) Vertreter: Uexküll & Stolberg

(57) **Zusammenfassung**

Es wird ein Verfahren zur Herstellung von mehrfarbigen Dentalrestaurationen beschrieben, bei dem Gläsern und Glaskeramiken mit unterschiedlichen Zusammensetzungen die Formen von Dentalrestaurationen gegeben werden und in den Gläsern und Glaskeramiken Farbänderungen bewirkt werden, indem diese mit künstlicher elektromagnetischer Strahlung bestrahlt und einer Wärmebehandlung unterzogen werden.

## Beschreibung

Die Erfindung betrifft ein Verfahren zur Herstellung einer mehrfarbigen Dentalrestauration, bei dem in einem Glas oder einer Glaskeramik eine Farbänderung bewirkt wird.

Glaskeramiken, wie z.B. Lithiumsilikat-Glaskeramiken, zeichnen sich typischerweise durch sehr gute mechanische Eigenschaften aus und finden deshalb als Material zur Herstellung von Dentalrestaurationen verbreitet Anwendung.

Verschiedene Methoden zur Formgebung, wie beispielsweise Pressverfahren, Sintern oder maschinelle Bearbeitung, werden eingesetzt, um Dentalrestaurationen aus Glaskeramik herzustellen. Als besonders vorteilhaft hat sich eine Formgebung mittels CAD/CAM-Verfahren erwiesen, bei denen zunächst ein digitales Modell der gewünschten Dentalrestauration in einem computergestützten Verfahren erstellt wird (Computer-aided Design). Auf Grundlage dieses Modells wird dann typischerweise durch maschinelle Bearbeitung, insbesondere durch Fräsen und Schleifen, in einem ebenfalls computergestützten Verfahren die gewünschte Dentalrestauration gefertigt (Computer-aided Manufacturing).

Glaskeramiken sind generell auch wegen ihrer vorteilhaften optischen Eigenschaften, wie beispielsweise einer hohen Transluzenz, für die Herstellung von Dentalrestaurationen geeignet.

Typischerweise wählt ein Zahnarzt oder ein Zahntechniker die für einen Patienten geeignete Farbe und Transluzenz aus einem Bestand an Rohlingen aus. Es ist für den Zahnarzt oder Zahntechniker allerdings nicht möglich, Rohlinge für alle natürlich auftretenden Farbwerte und Transluzenzstufen vorzuhalten. Jedoch sollte eine große Auswahl vorgehalten werden, um die Abweichungen in der Farbe und Transluzenz zwischen natürlichem Zahn und Dentalrestauration gering zu halten.

Natürliche Zähne weisen allerdings komplexe Farbgebungen mit Farbverläufen und 3D-Farbeffekten auf. Damit die Dentalrestauration im Gebiss eines Patienten nicht von dem umgebenden natürlichen Zahnmaterial zu unterscheiden ist, gilt es insbesondere im Bereich der Frontzähne, die komplexe Farbgebung der natürlichen Zähne für den jeweiligen Patienten möglichst naturgetreu zu imitieren.

Beispielsweise können einfarbigen Dentalrestaurationen durch eine nachträgliche Verblendung die gewünschten optischen Eigenschaften verliehen werden. Zum Beispiel kann im Rahmen der Cut-back-Technik zunächst aus einer vollanatomischen Krone ein Körper mit der Form eines natürlichen Dentinkerns gefräst werden. Auf diesen Körper, der für gewöhnlich eine geringe Transluzenz aufweist, können gegebenenfalls unterschiedlich gefärbte Materialschichten aufgetragen werden, die üblicherweise auch eine höhere Transluzenz aufweisen. Auf diese Weise kann eine Dentalrestauration erhalten werden, die hohen ästhetischen Ansprüchen genügt. Nachteilig ist jedoch, dass das Verfahren sehr aufwendig ist und zudem das Ergebnis stark von den Fertigkeiten des Zahntechnikers abhängig ist. Wünschenswert wäre es, ein einfacheres Farbgebungsverfahren bereitzustellen, welches eine individuelle Einfärbung der Dentalrestauration für hohe ästhetische Ansprüche ermöglicht. Das Verfahren sollte zudem schnell und effizient durchzuführen sein, automatisierbar sein, und das Ergebnis sollte weniger als bei bisher genutzten Verfahren von den handwerklichen Fertigkeiten des Zahntechnikers abhängen.

Es ist auch möglich, eine einfarbige Dentalrestauration so zu bemalen, dass die Farbgebung einem natürlichen Zahn ähnelt. Gleichsam wie die Cut-back-Technik ist auch bei diesem Verfahren das Ergebnis stark von den Fertigkeiten des Zahntechnikers abhängig. Nachteilig ist auch, dass beim Bemalen der Dentalrestauration für gewöhnlich keine 3D-Farbeffekte erzielt werden können, da die Farbe lediglich auf die Oberfläche der Restauration aufgetragen wird. Auch der typische Transluzenzverlauf natürlicher Zähne lässt sich durch Bemalen nicht imitieren. Aus diesen Gründen ist das ästhetische Ergebnis von bemalten Dentalrestaurationen vor allem bei solchen, die im Bereich der Frontzähne eingesetzt werden sollen, regelmäßig nicht zufriedenstellend.

In Hinblick auf die beschriebenen Schwierigkeiten sind verschiedene Verfahren zur Herstellung von mehrfarbigen Dentalrestaurationen mit naturgetreuer Farbgebung entwickelt worden.

Die EP 1 900 341 A1 beschreibt mehrfarbige Formkörper, die aus unterschiedlich gefärbten Schichten hergestellt sind, bei denen die Farbübergänge zwischen den Schichten nicht wahrnehmbar sind. Durch Trockenpressen von entsprechend gefärbten, aufeinander geschichteten Glaskeramik-Pulvern, Entbindern und Sintern werden mehrfarbige Formkörper hergestellt, aus denen im Rahmen von CAD/CAM-Verfahren mehrfarbige Dentalrestaurationen gefertigt werden können. Für die Herstellung einer individuell gefärbten Dentalrestauration muss allerdings in einem aufwendigen Verfahren ein individuell geschichteter Formkörper hergestellt werden.

Aus der EP 3 178 462 A1 ist ein Verfahren bekannt, bei dem eine mehrfarbige keramische Dentalrestauration dadurch hergestellt wird, dass ein poröser keramischer Körper zunächst mit einer Farbpigmentlösung beladen wird und die Farbpigmente dann in der Keramik durch Regelung eines oder mehrerer Umgebungsparameter, wie beispielsweise der Luftfeuchtigkeit und/oder des Druckes, ungleichmäßig verteilt werden. Die hergestellten Dentalkeramikrohlinge sind insbesondere zur Bearbeitung mittels CAD/CAM geeignet.

Die WO 2013/122662 beschreibt individuell gefärbte Fräsblöcke für den dentalen Einsatz und Verfahren zu deren Herstellung. Auf Basis der für die zu erstellende Dentalrestauration gewünschten Farbgebung wird ein Fräsblock in einem Rapid-Prototyping-Verfahren hergestellt. Dabei wird ein Material, das einem Härtungsschritt, beispielsweise durch Polymerisierung, unterzogen werden kann, schichtweise zu einem Fräsblock aufgebaut, wobei die einzelnen Schichten in den gewünschten Bereichen individuell eingefärbt werden können. Die bevorzugt verwendeten Materialien basieren typischerweise auf (Meth)acrylat-Verbundwerkstoffen und weisen nicht die vorteilhaften mechanischen Eigenschaften von Lithiumsilikat-Glaskeramiken auf.

Die EP 0 153 026 A1, DE 10 2005 003 595 A1, DE 103 04 382 A1, und US 2016/0340228 A1 beschreiben Verfahren zur Herstellung von Gläsern und Glaskeramiken, welche als optische Bauteile, wie Lichtleiter, eingesetzt werden. Es wird offenbart, dass insbesondere eine Behandlung der Gläser mit Licht und Wärme geeignet ist, um entweder die Brechungsindizes der Gläser zu verändern oder um Kristallisationskeime in den Gläsern zu bilden. Auch Einfärbungen sind beobachtet worden, welche für den Einsatz der Materialien als Lichtleiter allerdings unerwünscht sind. Es ist kein Hinweis zu finden, dass diese Verfahren Produkte ergeben könnten, welche die für den dentalen Einsatzbereich außergewöhnlich hohen Anforderungen an optische und mechanische Eigenschaften erfüllen könnten.

Ausgehend von den oben beschriebenen Nachteilen der bekannten Verfahren liegt der Erfindung die Aufgabe zugrunde, ein Verfahren zur Herstellung einer mehrfarbigen Dentalrestauration zur Verfügung zu stellen, bei dem eine Einfärbung auf einfachere Weise bewirkt werden kann. Darüber hinaus soll sich das Verfahren für die Herstellung von individuell gefärbten Dentalrestaurationen eignen, bei dem im eingesetzten Material gezielt Farbänderungen bewirkt werden, sodass die hergestellte Dentalrestauration Farbverläufe aufweist, die den Farbverläufen von natürlichem Zahnmaterial nachempfunden sind. Das Verfahren sollte automatisierbar sein und bevorzugt sollte es mit automatisierten Formgebungsverfahren kombinierbar sein. Die durch das Verfahren hergestellte mehrfarbige Dentalrestauration sollte ferner eine vorteilhafte Transluzenz und vorteilhafte mechanische Eigenschaften, wie beispielsweise eine hohe Festigkeit und Bruchzähigkeit, aufweisen sowie chemisch beständig und biokompatibel sein.

Diese Aufgabe wird durch das Verfahren nach den Ansprüchen 1 bis 17 gelöst. Die Erfindung ist ebenfalls auf die mehrfarbige Dentalrestauration nach Anspruch 18 sowie die Verwendungen nach den Ansprüchen 19 und 20 gerichtet.

Das erfindungsgemäße Verfahren zur Herstellung einer mehrfarbigen Dentalrestauration zeichnet sich dadurch aus, dass
a) einem Glas oder einer Glaskeramik die Form einer Dentalrestauration gegeben wird, und
b) in mindestens einem Teil des Glases oder der Glaskeramik eine Farbänderung bewirkt wird, indem dieser Teil mit künstlicher elektromagnetischer Strahlung bestrahlt und dieser bestrahlte Teil einer Wärmebehandlung unterzogen wird.

Es hat sich überraschend gezeigt, dass das erfindungsgemäße Verfahren die einfache Herstellung von mehrfarbigen Dentalrestaurationen ermöglicht, wobei die Mehrfarbigkeit innerhalb eines Glases oder einer Glaskeramik, wie z.B. eines Lithiumsilikatglases oder einer Lithiumsilikat-Glaskeramik, gezielt bewirkt werden kann. Es ist zudem überraschenderweise festgestellt worden, dass kontinuierliche Farbverläufe erzeugt werden können. Da anders als bei vielen herkömmlichen Verfahren nicht die Aufbringung farbgebender Materialien für die Farbänderung verantwortlich ist, sondern eine Farbänderung in dem Glas und der Glaskeramik bewirkt wird, können sogar 3D-Farbverläufe erzielt werden.

Das Verfahren ermöglicht zudem überraschenderweise eine einfache sowie automatisierbare und individualisierbare Farbwahl und Farbgebung. Es kann bekannte automatisierte Verfahren, wie beispielsweise CAD/CAM-Verfahren, zur Formgebung einschließen, wodurch eine umfassende Digitalisierung der Gestaltung und Herstellung von individuell geformten und individuell gefärbten Dentalrestaurationen erreicht werden kann. Durch solch einen hohen Grad an Digitalisierung und Automatisierung wird im Vergleich zu herkömmlichen Verfahren, wie beispielsweise dem Bemalen von Dentalrestaurationen oder dem Cut-back-Verfahren, eine schnelle und präzise Herstellung ermöglicht, bei der die Ästhetik des Ergebnisses wenig von den handwerklichen Fähigkeiten der mit der Herstellung betrauten Person abhängt.

Das Verfahren ist auch in Bezug auf die Lagerung und Logistik der Ausgangsmaterialien beim Zahnarzt oder Zahntechniker vorteilhaft. Erfindungsgemäß kann aus wenigen unterschiedlichen Ausgangsmaterialien, insbesondere wenigen Typen von Rohlingen, die gewünschte Einfärbung von Dentalrestaurationen erzielt werden. Es ist demnach nicht länger notwendig, dass ein Zahnarzt oder Zahntechniker Rohlinge oder Rohmaterialien in vielen handelsüblich verfügbaren Farbwerten und Transluzenzstufen vorhält oder jeweils auf Bedarf bestellt. Für den gewöhnlichen Bedarf eines Zahnarztes oder eines Zahntechnikers kann es vielmehr ausreichend sein, Rohlinge mit nur einem Farbwert in verschiedenen Transluzenzstufen vorzuhalten.

Eine erfindungsgemäß bewirkte "Farbänderung" liegt vor, wenn sich ein bestrahlter und wärmebehandelter Teil des Glases oder der Glaskeramik nach der Durchführung von Schritt b) von dem Teil vor der Durchführung von Schritt b) in mindestens einem von Farbwert, Helligkeit und Transluzenz, vorzugsweise in mindestens einem von Farbwert und Helligkeit, unterscheidet. Die Begriffe "Farbe" und "gefärbt" beziehen sich also auf den Farbwert, die Helligkeit und die Transluzenz eines Materials.

Farbwerte und Helligkeiten können durch den L*a*b-Wert, insbesondere bestimmt nach DIN 6174, oder durch einen in der Dentalindustrie gängigen Farbschlüssel charakterisiert werden. Die Farbmessung kann mit handelsüblichen Messgeräten, wie einem Spektralphotometer CM-3700d (Konica Minolta), durchgeführt werden. Beispiele für Farbschlüssel sind der Vitapan classical® und der Vita 3D Master®, beide von der VITA Zahnfabrik H. Rauter GmbH & Co. KG, und der Chromascop® der Ivoclar Vivadent AG.

Die "Transluzenz" ist die Lichtdurchlässigkeit eines Materials, d.h. das Verhältnis von durchgelassener zu einfallender Lichtintensität. Die Transluzenz kann in Form des Kontrastwerts (CR-Wert) gemäß dem British Standard 5612 bestimmt werden.

Durch das erfindungsgemäße Verfahren kann im Wesentlichen jede für die Herstellung von Dentalrestaurationen gewünschte Farbe bewirkt werden. Insbesondere können gewünschte Gelbtöne (b*-Werte) und Rottöne (a*-Werte) gezielt bewirkt werden. Verfahren zur Festlegung gewünschter Materialfarben bei der Herstellung von Dentalrestaurationen sind aus dem Stand der Technik, z.B. aus der WO 2018/162671, bekannt.

Es ist bevorzugt, dass eine bei der Farbänderung gemäß Schritt b) erzielte Farbe einen b*-Wert von mindestens 4,00 und einen a*-Wert von mindestens -1,00, bestimmt nach DIN 6174, aufweist.

Es ist ferner möglich, die Farbänderung gemäß Schritt b) des erfindungsgemäßen Verfahrens mit anderen farbgebenden Verfahren zur Herstellung von Dentalrestaurationen, wie z.B. Ionenfärbung oder der Verwendung färbender Pigmente, zu kombinieren.

Es ist besonders bevorzugt, dass das im erfindungsgemäßen Verfahren eingesetzte Glas und die Glaskeramik mindestens eine oxidierbare Komponente und mindestens eine reduzierbare Färbekomponente enthalten.

Der Begriff "oxidierbare Komponente" bezeichnet eine Komponente, die durch Bestrahlung des Glases und der Glaskeramik in Schritt b) oxidiert werden kann. Bevorzugte oxidierbare Komponenten sind Cer-Ionen, Europium-Ionen, Erbium-Ionen, Kupfer-Ionen und deren Mischungen, insbesondere Cer-Ionen. Besonders bevorzugt ist Ce³⁺ die oxidierbare Komponente.

In einer bevorzugten Ausführungsform beinhaltet das erfindungsgemäße Verfahren, dass das Glas und die Glaskeramik Ce, berechnet als CeO₂, bevorzugt in einer Menge von 0,01 bis 1,5, insbesondere 0,03 bis 1 Gew.-%, enthalten. "Ce" bezeichnet erfindungsgemäß alle im Glas und in der Glaskeramik vorliegenden Oxidationsstufen von Cer.

Ce kann mittels UV/Vis-Spektroskopie nachgewiesen werden. Eine Quantifizierung des Ce³⁺-Gehalts ist mittels Fluoreszenzspektroskopie möglich. Unter Berücksichtigung der zur Glasherstellung eingesetzten Mengen an Ce und des ermittelten Ce³⁺-Gehalts kann der Ce⁴⁺-Gehalt des Glases und der Glaskeramik bestimmt werden.

Der Begriff "reduzierbare Färbekomponente" bezeichnet eine Komponente, die unter Ausbildung einer Farbänderung reduziert werden kann. Bevorzugte reduzierbare Färbekomponenten sind Kationen von Metallen, wie beispielsweise von Ag, Au, Cu, oder deren Kombinationen, besonders bevorzugt Kationen von Ag und/oder Au.

Die Metalle, wie z.B. Ag und Au, können in unterschiedlichen Oxidationsstufen im Glas und in der Glaskeramik vorhanden sein. Ohne eine nähere Bestimmung beziehen sich die Bezeichnungen "Ag" und "Au" erfindungsgemäß auf alle im Glas und in der Glaskeramik vorliegenden Oxidationsstufen dieser Metalle, bevorzugt auf die Oxidationsstufen 0 und I.

In einer bevorzugten Ausführungsform des erfindungsgemäßen Verfahrens enthalten das Glas und die Glaskeramik Ag, berechnet als Ag₂O, bevorzugt in einer Menge von 0,0005 bis 1,3, insbesondere 0,002 bis 0,7 Gew.-%.

In einer weiteren bevorzugten Ausführungsform enthalten das Glas und die Glaskeramik Au, berechnet als Au₂O, bevorzugt in einer Menge von 0,0001 bis 0,65, insbesondere 0,0003 bis 0,25, besonders bevorzugt 0,003 bis 0,2 Gew.-%.

In einer besonders bevorzugten Ausführungsform des erfindungsgemäßen Verfahrens enthalten das Glas und die Glaskeramik Ce sowie Ag und/oder Au.

Sind Ce und Ag im Glas und in der Glaskeramik enthalten, kann in Schritt b) insbesondere eine Gelbfärbung bewirkt werden. Sind Ce und Au im Glas und in der Glaskeramik enthalten, kann in Schritt b) insbesondere eine Rotfärbung bewirkt werden. Sind Ce, Ag und Au im Glas und in der Glaskeramik enthalten, kann in Schritt b) insbesondere eine Gelb- und eine Rotfärbung bewirkt werden.

Die Mengen von Ce, Ag und Au im Glas und in der Glaskeramik, insbesondere die Mengen von Ag und Au, können die durch die Farbänderung erzielte Farbe beeinflussen. Es wurde festgestellt, dass durch die Verwendung einer größeren Menge an Ag und/oder Au regelmäßig eine intensivere Farbe bewirkt werden kann.

Ohne Beschränkung auf eine Theorie wird angenommen, dass die Bestrahlung in Schritt b) eine Redoxreaktion ermöglicht, bei der die oxidierbare Komponente oxidiert und die reduzierbare Färbekomponente reduziert werden kann.

Sind z.B. Ce und Ag im Glas und in der Glaskeramik enthalten, wird angenommen, dass die elektromagnetische Strahlung eine Reaktion gemäß der folgenden Gleichung ermöglicht:

Ce³⁺ + Ag⁺ → Ce⁴⁺ + Ag⁰

Es hat sich gezeigt, dass die Wärmebehandlung des bestrahlten Glases oder der bestrahlten Glaskeramik von Schritt b) dazu führen kann, dass Partikel mit reduzierter Färbekomponente entstehen oder solche Partikel, die bereits bestehen, anwachsen. Diese Partikel können insbesondere als "Cluster" oder "Kolloid" vorliegen und zur Farbe des Glases und der Glaskeramik beitragen. Solche Partikel können z.B. mittels Transmissionselektronenmikroskopie (TEM) nachgewiesen werden.

Es wurde z.B. festgestellt, dass Partikel mit Ag typischerweise eine Gelbfärbung und Partikel mit Au typischerweise eine Rotfärbung im Glas und in der Glaskeramik bewirken.

In einer weiteren bevorzugten Ausführungsform enthalten das Glas und die Glaskeramik, in denen in Schritt b) eine Farbänderung bewirkt wird, Ag-Halogenide, wie z.B. AgCl, AgBr und/oder AgI. Derartige Ag-Halogenide können in der Regel qualitativ, beispielsweise mittels NMR-Spektroskopie, Transmissionselektronenmikroskopie oder Raman-Spektroskopie, nachgewiesen werden.

In einer bevorzugten Ausführungsform enthalten das Glas und die Glaskeramik, Cl, Br und/oder I, insbesondere Cl, in einer Menge von 0,0001 bis 0,9, besonders bevorzugt 0,0005 bis 0,7 Gew.-%. In der Regel liegen Cl, Br und I im Glas und in der Glaskeramik in ionischer Form, bevorzugt in der Oxidationsstufe -I, vor. Diese Halogenide stammen üblicherweise von als Ausgangsmaterialien verwendeten Halogeniden, wie Ag-Halogeniden oder Cu-Halogeniden.

In einer weiteren bevorzugten Ausführungsform enthalten das Glas und die Glaskeramik, in denen in Schritt b) des Verfahrens eine Farbänderung bewirkt wird, 0,01 bis 1,5 Gew.-% Ce, berechnet als CeO₂, 0,0005 bis 1,3 Gew.-% Ag, berechnet als Ag₂O, 0,06 bis 0,5 Gew.-% Sb, berechnet als Sb₂O₃, sowie 0,01 bis 0,15 Gew.-% Sn, berechnet als SnO. Diesem bevorzugten Glas und dieser bevorzugten Glaskeramik kann eine Gelb- und eine Rotfärbung verliehen werden, wobei typischerweise ein höherer Gehalt an Sb eine intensivere Rotfärbung bewirkt.

Zum Bewirken einer Farbänderung werden das Glas und die Glaskeramik zunächst in Schritt b) mit künstlicher elektromagnetischer Strahlung bestrahlt. Als "künstliche elektromagnetische Strahlung" ist erfindungsgemäß eine von einer künstlichen Strahlungsquelle emittierte Strahlung zu verstehen.

Die Strahlung, mit der das Glas und die Glaskeramik bestrahlt werden, kann Anteile mit unterschiedlichen Wellenlängen enthalten. Für das erfindungsgemäße Verfahren ist es ausreichend, wenn ein Anteil der gesamten Strahlung für das Bewirken einer Farbänderung geeignet ist.

In einer bevorzugten Ausführungsform werden das Glas und die Glaskeramik mit Röntgenstrahlung bestrahlt. Die verwendete Röntgenstrahlungsquelle weist vorzugsweise ein Anodenmaterial ausgewählt aus der Gruppe bestehend aus Cu, Co, Cr, Fe oder Mo, insbesondere Cu, auf. Die Bestrahlung kann mit Röntgenenergien unterschiedlicher Spektrallinien erfolgen. Vorzugsweise erfolgt die Bestrahlung mit K-α-Strahlung.

In einer weiteren bevorzugten Ausführungsform werden das Glas und die Glaskeramik mit UV-Strahlung bestrahlt. Erfindungsgemäß bezeichnet "UV-Strahlung" eine elektromagnetische Strahlung mit einer Wellenlänge von 100 bis 400 nm.

In einer besonders bevorzugten Ausführungsform hat die Strahlung eine Wellenlänge von höchstens 380 nm, insbesondere im Bereich von 100 bis 360 nm, besonders bevorzugt im Bereich von 250 bis 350 nm, am meisten bevorzugt im Bereich von 300 bis 310 nm.

Es ist besonders bevorzugt, dass die Strahlung von einer Strahlungsquelle emittiert wird, die im Wellenlängenbereich von 180 bis 400 nm eine effektive Bestrahlungsstärke von mehr als 1 mW/m², insbesondere mehr als 3 mW/m², besonders bevorzugt mehr als 10 mW/m², am meisten bevorzugt mehr als 100 mW/m², bestimmt in einem Abstand von 20 cm von der Strahlungsquelle als spektral gewichtete Bestrahlungsstärke nach DIN EN 62471:2009-03, hat.

Es ist bevorzugt, in Schritt b) eine UV-LED, einen UV-Laser, ein Röntgendiffraktometer oder eine Quecksilberdampflampe als Strahlungsquelle zu verwenden.

In einer bevorzugten Ausführungsform ist die Wellenlänge der Strahlung an die im Glas und in der Glaskeramik vorliegende oxidierbare Komponente angepasst. Es hat sich z.B. gezeigt, dass eine Wellenlänge im Bereich von etwa 310 nm vorteilhaft ist, wenn Cer-Ionen im Glas und in der Glaskeramik enthalten sind. Für Kupfer-Ionen ist hingegen eine Wellenlänge von etwa 280 nm vorteilhaft.

Es wurde festgestellt, dass die gemäß Schritt b) erzielte Farbänderung in der Regel von den Bedingungen der Bestrahlung, insbesondere Bestrahlungsdauer, Bestrahlungsstärke und Wellenlänge der Strahlung, abhängig ist. Insbesondere wurde festgestellt, dass typischerweise mittels einer höheren Bestrahlungsstärke oder einer längeren Bestrahlungsdauer eine intensivere Farbe bewirkt werden kann.

In einer Ausführungsform werden in Schritt b) das gesamte Glas oder die gesamte Glaskeramik bestrahlt. Zur Erzielung von Mehrfarbigkeit werden dabei in der Regel verschiedene Bereiche unter unterschiedlichen Bedingungen bestrahlt.

In einer bevorzugten Ausführungsform werden in Schritt b) nur Teile des Glases oder der Glaskeramik bestrahlt. Obwohl eine Bestrahlung von nur Teilen dem Glas oder der Glaskeramik bereits Mehrfarbigkeit verleiht, können auch in diesem Fall verschiedene Bereiche unter unterschiedlichen Bedingungen bestrahlt werden.

Bei der Bestrahlung des Glases und der Glaskeramik können kleine Durchmesser des Strahls vorteilhaft sein, um die Farbänderung auf kleine Bereiche des Glases oder der Glaskeramik zu begrenzen und auf diese Weise feine Farbverläufe des natürlichen Zahnmaterials präzise zu imitieren. Große Durchmesser des Strahls können hingegen für eine gleichmäßige Bestrahlung des Glases oder der Glaskeramik vorteilhaft sein und dazu beitragen, den Zeitaufwand des Verfahrens gering zu halten.

In einer bevorzugten Ausführungsform weist die Strahlung einen definierten lokalen Fokus auf. Besonders bevorzugt kann dieser Fokus auch auf Bereiche des Glases und der Glaskeramik gerichtet werden, die nicht in an der Oberfläche des Glases und der Glaskeramik liegen. Durch eine Fokussierung der Strahlung oder durch die Verwendung von mehreren Strahlengängen mit definierten Fokussen kann erreicht werden, dass die Farbänderung im Glas und der Glaskeramik vorwiegend im fokussierten Bereich bewirkt wird, wohingegen entlang des Strahlengangs im Glas und in der Glaskeramik keine oder kaum eine Farbänderung bewirkt wird.

Um die Bestrahlung auf bestimmte Bereiche des Glases und der Glaskeramik zu beschränken und/oder die Strahlung abzuschwächen, können bei der Bestrahlung in Schritt b) Schablonen verwendet werden.

Zum Bewirken einer Farbänderung wird in Schritt b) des erfindungsgemäßen Verfahrens mindestens ein bestrahlter Teil des Glases oder der Glaskeramik wärmebehandelt. Die Wärmebehandlung erfolgt bevorzugt bei einer Temperatur im Bereich von 300 bis 1000°C, insbesondere 400 bis 950°C, besonders bevorzugt 450 bis 850°C.

In einer bevorzugten Ausführungsform erfolgt die Wärmebehandlung für eine Dauer von bis zu 120 min, insbesondere bis zu 60 min.

Es hat sich gezeigt, dass die bei der Farbänderung gemäß Schritt b) erzielte Farbe in der Regel von den Bedingungen der Wärmebehandlung abhängig ist. Es wurde z.B. festgestellt, dass mittels einer höheren Temperatur oder einer längeren Dauer in der Regel eine intensivere und/oder dunklere Farbe bewirkt werden kann. Dabei stehen die Temperatur und die Dauer, die zum Erreichen einer bestimmten Farbänderung notwendig sind, regelmäßig miteinander in Wechselwirkung. Im Allgemeinen gilt, dass bei einer erhöhten Temperatur die Dauer für das Bewirken einer bestimmten Farbänderung verkürzt werden kann, und umgekehrt.

Typischerweise werden das Glas und die Glaskeramik in einem Ofen der Wärmebehandlung in Schritt b) unterzogen. Geeignete Öfen sind z.B. Öfen vom Typ Programat der Ivoclar Vivadent AG.

In einer weiteren bevorzugten Ausführungsform erfolgt die Wärmebehandlung in Schritt b) mit einem Laser, wie beispielsweise einem UV-Laser, VIS-Laser oder einem IR-Laser, insbesondere mit einem IR-Laser, der Strahlung mit einer Wellenlänge größer als 5 µm erzeugt, oder einem VIS-Laser, der Strahlung mit einer Wellenlänge von 500 bis 600 nm, besonders bevorzugt von 515 bis 532 nm, erzeugt. Die Laserparameter (Wellenlänge, Pulsdauer, Pulsenergie, Dauerstrichbetrieb) werden üblicherweise so gewählt, dass das Glas und die Glaskeramik auf die für die Farbänderung erforderliche Temperatur erhitzt werden, jedoch nicht verdampft oder geschädigt werden.

Eine Wärmebehandlung eines Glases und einer Glaskeramik mit einem Laser erfolgt in der Regel nur in ausgewählten Bereichen des Glases und der Glaskeramik, wodurch die Farbänderung auf ausgewählte Bereiche begrenzt oder verschiedene Bereiche des Glases und der Glaskeramik in einem unterschiedlichen Ausmaß einer Wärmebehandlung unterzogen werden können. Die Durchführung der Wärmebehandlung mit einem Laser kann besonders vorteilhaft sein, um komplizierte dreidimensionale Farbverläufe zu bewirken oder die Transluzenz des Materials zu verändern.

In einer bevorzugten Ausführungsform bewirkt die Wärmebehandlung des bestrahlten Glases und der bestrahlten Glaskeramik in Schritt b) auch eine Kristallisation im Glas oder eine weitere Kristallisation in der Glaskeramik, wie z.B. die Kristallisation von Lithiummetasilikat und/oder Lithiumdisilikat. Damit kann die gesamte Verfahrensdauer zur Herstellung der Dentalrestauration auf vorteilhafte Weise verkürzt werden.

In einer weiteren Ausführungsform erfolgt die Wärmebehandlung in Schritt b) zusätzlich zu Wärmebehandlungen, die zur Bildung gewünschter Kristalle durchgeführt werden.

Die Bestrahlung und die Wärmebehandlung in Schritt b) können in einem Schritt oder in getrennten Schritten durchgeführt werden.

Bestrahlung und Wärmebehandlung werden typischerweise in getrennten Verfahrensschritten durchgeführt. In diesem Fall werden das Glas und die Glaskeramik mit einer ersten Strahlungsquelle bestrahlt und das bestrahlte Glas und die bestrahlte Glaskeramik werden in einem nachfolgenden Schritt, insbesondere mithilfe einer anderen Vorrichtung, wie einer zweiten Strahlungsquelle oder eines Ofens, einer Wärmebehandlung unterzogen.

Es kann jedoch auch vorteilhaft sein, die Bestrahlung und die Wärmebehandlung in einem Schritt durchzuführen. In diesen Fall können die Bestrahlung und die Wärmebehandlung gleichzeitig durch unterschiedliche Vorrichtungen erfolgen. Vorzugsweise werden dabei ein UV-Laser für die Bestrahlung und ein Ofen, ein VIS- oder ein UV-Laser für die Wärmebehandlung verwendet. Die Bestrahlung und die Wärmebehandlung können jedoch auch mit einer Vorrichtung durchgeführt werden. Dafür ist typischerweise eine Bestrahlung des Glases und der Glaskeramik mit einer derartig hohen Bestrahlungsstärke notwendig, dass die durch die Strahlung bewirkte Wärmeentwicklung im Glas und in der Glaskeramik ausreicht, um eine Farbänderung zu erzielen.

Erfolgen Bestrahlung und Wärmebehandlung in getrennten Verfahrensschritten, kann die Wärmebehandlung unmittelbar nach der Bestrahlung durchgeführt werden.

In einer weiteren bevorzugten Ausführungsform wird die Wärmebehandlung hingegen in einem Schritt durchgeführt, der nicht unmittelbar an die Bestrahlung anschließt. Die Farbänderung nach Schritt b) des erfindungsgemäßen Verfahrens kann auch dadurch bewirkt werden, dass das bestrahlte Glas oder die bestrahlte Glaskeramik gelagert wird und zu einem späteren Zeitpunkt, z.B. nach mehreren Tagen oder Wochen, einer Wärmebehandlung unterzogen wird.

Ein für das erfindungsgemäße Verfahren geeignetes Glas und eine geeignete Glaskeramik werden üblicherweise aus einer entsprechenden Mischung von geeigneten Ausgangsmaterialien, wie z.B. Carbonaten, Oxiden, Phosphaten und Halogeniden, hergestellt.

Es ist bevorzugt, als Ce-haltige Rohstoffe Ce-Oxide, Ce-Carbonate, Ce-Halogenide, Ce-Sulfate und/oder Ce-Phosphate zu verwenden. In einer besonders bevorzugten Ausführungsform wird CeO₂, CeCl₃, CeF₃, CeI₃, CeBr₃, Ce₂(SO₄)₃ oder eine Kombination davon als Ce-haltiger Rohstoff verwendet.

Es ist bevorzugt, dass das Glas und die Glaskeramik P₂O₅, insbesondere in einer Menge von 0,5 bis 11,0 Gew.-%, besonders bevorzugt 0,9 bis 8,0 Gew.-%, enthalten. Mit einem Keimbildner, wie z.B. P₂O₅, läuft die Kristallisation, bei der das Glas in eine Glaskeramik umgewandelt wird, regelmäßig über den Mechanismus der Volumenkristallisation ab. Im Glas vorhandener Keimbildner ist daher bevorzugt homogen darin verteilt. Weitere mögliche Keimbildner sind TiO₂, ZrO₂, Nb₂O₅, Metalle, z.B. Pt, Pd, Ag und Au, oder Mischungen davon.

Es ist bevorzugt, dass das Glas und die Glaskeramik mindestens eine und bevorzugt alle der folgenden Komponenten in den angegebenen Mengen enthalten:

| Komponente | Gew.-% |
|---|---|
| SiO₂ | 61,0 - 88,0 |
| Li₂O | 5,0 - 24,0 |
| Al₂O₃ | 0 - 14,0 |
| P₂O₅ | 0,5 - 11,0 |
| Ce, berechnet als CeO₂ | 0,01 - 1,5 |
| Ag, berechnet als Ag₂O | 0,0005 - 1,3 |
| Au, berechnet als Au₂O | 0,0001 - 0,65 |

Besonders bevorzugt enthalten das Glas und die Glaskeramik mindestens eine und bevorzugt alle der folgenden Komponenten in den angegebenen Mengen:

| Komponente | Gew.-% |
|---|---|
| SiO₂ | 66,0 - 81,0 |
| Li₂O | 6,0 - 22,0 |
| Al₂O₃ | 1,0 - 12,0 |
| P₂O₅ | 0,9 - 8,0 |
| Ce, berechnet als CeO₂ | 0,03 - 1,0 |
| Ag, berechnet als Ag₂O | 0,002 - 0,7 |
| Au, berechnet als Au₂O | 0,0003 - 0,25 |

In einer Ausführungsform enthalten das Glas und die Glaskeramik neben Li₂O weiteres Alkalimetalloxid Me^{I}₂O in einer Menge von 0 bis 13,0, bevorzugt 0 bis 12,0 und besonders bevorzugt 1,0 bis 11,0 Gew.-%, wobei Me^{I}₂O ausgewählt ist aus K₂O, Na₂O, Rb₂O und/oder Cs₂O.

Des Weiteren ist es bevorzugt, dass das Glas und die Glaskeramik 0 bis 6,0 und bevorzugt 0 bis 5,0 Gew.-% weiteres Oxid zweiwertiger Elemente Me^{II}O enthalten, wobei Me^{II}O ausgewählt ist aus MgO, CaO, SrO, und/oder ZnO.

Es ist weiter bevorzugt, dass das Glas und die Glaskeramik 0 bis 2,0 und bevorzugt 0 bis 1,0 Gew.-% weiteres Oxid dreiwertiger Elemente Me^{III}₂O₃ enthalten, wobei Me^{III}₂O₃ ausgewählt ist aus B₂O₃, Y₂O₃, La₂O₃ und/oder Er₂O₃.

Ferner ist es bevorzugt, dass das Glas und Glaskeramik 0 bis 2,0 Gew.-% und bevorzugt 0 bis 1,0 Gew.-% weiteres Oxid vierwertiger Elemente Me^{IV}O₂ enthalten, wobei Me^{IV}O₂ ausgewählt ist aus SnO₂, ZrO₂, und/oder GeO₂.

Außerdem ist es bevorzugt, dass das Glas und die Glaskeramik 0 bis 2,0 Gew.-% und bevorzugt 0 bis 1,0 Gew.-% weiteres Oxid fünfwertiger Elemente Me^{V}₂O₅ enthalten, wobei Me^{V}₂O₅ ausgewählt ist aus V₂O₅, Ta₂O₅ und/oder Nb₂O₅.

Auch ist es bevorzugt, dass das Glas und die Glaskeramik 0 bis 7,5 und bevorzugt 0 bis 6,5 Gew.-% Oxid sechswertiger Elemente Me^{VI}O₃ enthalten, wobei Me^{VI}O₃ ausgewählt ist aus MoO₃ und/oder WO₃.

Manche der vorstehend genannten Komponenten können als Färbemittel und/oder Fluoreszenzmittel dienen. Das Glas und die Glaskeramik können auch noch zusätzliche Färbe- und Fluoreszenzmittel, wie beispielsweise farbgebende Metalloxide und/oder handelsübliche isochrome Farbkörper, enthalten.

Typischerweise wird zur Herstellung des Glases eine entsprechende Mischung von geeigneten Ausgangsmaterialien bei Temperaturen von insbesondere 1000 bis 1800°C, bevorzugt bei etwa 1400 bis 1650°C, für eine Dauer von 0,5 bis 10 h erschmolzen und dann abgekühlt.

Um eine hohe Homogenität zu erzielen, kann die erhaltene Glasschmelze in Wasser gegossen werden, um ein Glasgranulat zu bilden. Das Granulat kann dann erneut aufgeschmolzen werden. Die Schmelze kann in Formen gegossen werden, um Rohlinge des Glases, sogenannte Massivglasrohlinge oder monolithische Rohlinge, zu erzeugen.

Das Abkühlen kann in kontrollierter Weise durchgeführt werden, um eine Entspannung des Glases zu ermöglichen und Spannungen in der Struktur zu vermeiden, die mit schnellen Temperaturänderungen verbunden sind. In der Regel wird die Schmelze dafür in vorgewärmte Formen z.B. bei einer Temperatur von 400°C gegossen oder langsam in einem Ofen abgekühlt.

Im erfindungsgemäßen Verfahren eingesetzte Gläser können Keime zur Bildung von Kristallphasen enthalten, was auch als "keimhaltiges Glas" bezeichnet wird, und sind in der Regel Vorläufer der entsprechenden Glaskeramiken. Beispielsweise können Lithiumsilikatgläser Keime zur Bildung von Lithiummetasilikat-Kristallen und/oder Lithiumdisilikat-Kristallen enthalten.

Aus einem erzeugten Glas kann mittels Wärmebehandlungen eine erfindungsgemäß verwendete Glaskeramik hergestellt werden. Typischerweise umfasst die Herstellung von Glaskeramiken mehrere Wärmebehandlungen für die Keimbildung und Kristallisation.

Für die Herstellung einer Lithiumsilikat-Glaskeramik wird in der Regel z.B. durch eine erste Wärmebehandlung eine Ausbildung von Kristallisationskeimen bewirkt, die zur Bildung von Lithiummetasilikat-Kristallen geeignet sind. Meist erfolgen eine zweite Wärmebehandlung zur Kristallisation von Lithiummetasilikat und eine dritte Wärmebehandlung zur Umwandlung von Lithiummetasilikat in Lithiumdisilikat. Es ist möglich, mehrere Schritte, wie z.B. Keimbildung und Kristallisation von Lithiummetasilikat im Rahmen von nur einer Wärmebehandlung zu bewirken. Im Stand der Technik, beispielsweise aus der DE 103 36 913 A1, sind verschiedene geeignete Bedingungen, d.h. insbesondere geeignete Temperaturbereiche, Aufheizgeschwindigkeiten und Behandlungsdauern, zur Herstellung von Lithiumsilikat-Glaskeramiken bekannt.

Es ist bevorzugt, dass das Glas und die Glaskeramik, in denen in Schritt b) eine Farbänderung bewirkt wird, ausgewählt sind aus der Gruppe bestehend aus Lithiumsilikatglas, Lithiumalumosilikatglas, Lithiumsilikat-Glaskeramik, Lithiumalumosilikat-Glaskeramik und Quarz-Glaskeramik.

Die in der Glaskeramik enthaltenen Kristallphasen können durch Röntgenbeugungsanalyse (XRD) bestimmt werden. Die Bestimmung der Massen der Kristallphasen kann insbesondere nach der Rietveld-Methode erfolgen. Ein dafür geeignetes Verfahren ist z.B. in der Dissertation von M. Dittmer "Gläser und Glaskeramiken im System MgO-Al₂O₃-SiO₂ mit ZrO₂ als Keimbildner", Universität Jena 2011, beschrieben.

In einer bevorzugten Ausführungsform des erfindungsgemäßen Verfahrens enthält die in Schritt b) zu bestrahlende Glaskeramik Lithiummetasilikat, Lithiumdisilikat, Tiefquarz oder Lithiumalumosilikat als Hauptkristallphase.

Dabei bezeichnet der Begriff "Hauptkristallphase" die Kristallphase, die von allen in der Glaskeramik vorhandenen Kristallphasen den höchsten Massenanteil hat.

In einer bevorzugten Ausführungsform enthält die Glaskeramik mehr als 5 Gew.-%, bevorzugt mehr als 10 Gew.-% und besonders bevorzugt mehr als 20 Gew.-% Lithiummetasilikat, bezogen auf die gesamte Glaskeramik.

In einer weiteren bevorzugten Ausführungsform enthält die Glaskeramik mehr als 10 Gew.-%, bevorzugt mehr als 30 Gew.-.-% und besonders bevorzugt mehr als 50 Gew.-% Lithiumdisilikat, bezogen auf die gesamte Glaskeramik.

In einer bevorzugten Ausführungsform des erfindungsgemäßen Verfahrens enthält die in Schritt b) zu bestrahlende Glaskeramik neben Lithiummetasilikat und/oder Lithiumdisilikat eine oder mehrere weitere Kristallphasen ausgewählt aus Lithiumalumosilikat, Lithiumorthophosphat, Apatit, Tiefquarz, Cristobalit, Diopsid, Wollastonit, Scheelit und Powellit, besonders bevorzugt Tiefquarz und/oder Lithiumalumosilikat.

In Schritt a) des erfindungsgemäßen Verfahrens werden dem Glas und der Glaskeramik die Form einer gewünschten Dentalrestauration gegeben. Es ist besonders bevorzugt, dass dem Glas und der Glaskeramik in Schritt a) durch Verpressen oder maschinelle Bearbeitung die Form der Dentalrestauration gegeben werden.

Die Formgebung kann umfassen, dass das Glas und die Glaskeramik, beispielsweise in Form eines teilweise vorgesinterten Lithiumsilikat-Rohlings, bei erhöhter Temperatur und erhöhtem Druck zu einer gewünschten Form verpresst werden. Beim Verpressen wird das eingesetzte Material in einen viskosen Zustand überführt, sodass es unter dem Einfluss des erhöhten Drucks in die gewünschte Form fließen kann.

In einer bevorzugten Ausführungsform erfolgt die Formgebung in Schritt a) durch maschinelle Bearbeitung. Die maschinelle Bearbeitung erfolgt typischerweise durch materialabtragende Verfahren, wie beispielsweise durch Fräsen und Schleifen. Für die maschinelle Bearbeitung werden bevorzugt Glaskeramiken, insbesondere Lithiumsilikat-Glaskeramiken, verwendet, die Lithiummetasilikat oder Lithiumdisilikat, ganz besonders bevorzugt Lithiummetasilikat, als Hauptkristallphase enthalten. Die Verwendung von nicht überwiegend zu Lithiumdisilikat kristallisierter Lithiumsilikat-Glaskeramik bietet den Vorteil, dass eine leichtere maschinelle Bearbeitung mit geringerem Materialverschleiß möglich ist. Nach der maschinellen Bearbeitung eines solchen teilkristallisierten Materials wird dieses typischerweise einer Wärmebehandlung unterzogen, um eine weitere Kristallisation, vorzugsweise zu Lithiumdisilikat, zu bewirken.

Es ist bevorzugt, dass die maschinelle Bearbeitung in einem CAD/CAM-Verfahren erfolgt. Dabei können Glaskeramiken, wie Lithiumsilikat-Glaskeramiken, insbesondere in Form von Rohlingen, eingesetzt werden. Die Form dieser Rohlinge kann an den Typ der für die maschinelle Bearbeitung eingesetzten Maschine angepasst sein.

In einer weiteren bevorzugten Ausführungsform des Verfahrens wird der Farbänderungsschritt mit einem CAD/CAM-Verfahren kombiniert. Das erfindungsgemäße Verfahren schließt somit Verfahren ein, bei denen im CAD-Schritt zusätzlich Informationen über die farbliche Gestaltung des Gebisses aufgenommen und verarbeitet werden. Auf Basis eines 3D-Modells, das auch die verarbeiteten Farbinformationen berücksichtigt, kann eine Dentalrestauration modelliert werden, die nicht nur die gewünschte Form, sondern auch die gewünschte farbliche Gestaltung aufweist. Auf Basis dieses Modells kann im CAM-Schritt eine Dentalrestauration gefertigt werden. Dabei werden das Glas und die Glaskeramik, bevorzugt in Form eines Rohlings, typischerweise durch maschinelle Bearbeitung geformt und gemäß Schritt b) bestrahlt und einer Wärmebehandlung unterzogen. Vorzugsweise erfolgt die Bestrahlung in Bereichen, die bereits die Form der gewünschten Dentalrestauration aufweisen. Es kann jedoch auch wünschenswert sein, die maschinelle Bearbeitung zur Formgebung durchzuführen, nachdem die Farbänderung gemäß Schritt b) bewirkt worden ist.

Dentalrestaurationen mit individueller Form und individueller Farbgebung können somit in einem automatisierten Verfahren hergestellt werden. Solch ein CAD/CAM-Verfahren mit Farbgebung ist sehr attraktiv, da es ermöglicht, den Patienten schnell mit der gewünschten Dentalrestauration zu versorgen. Genau wie bei konventionellen CAD/CAM-Verfahren ist eine sogenannte Chairside-Behandlung (Behandlung am Patientenstuhl) für den Zahnarzt möglich.

In einer besonders bevorzugten Ausführungsform des erfindungsgemäßen Verfahrens ist die Dentalrestauration eine Brücke, ein Inlay, ein Onlay, ein Veneer, ein Abutment, eine Teilkrone, eine Krone oder eine Schale.

Die Schritte a) und b) des erfindungsgemäßen Verfahrens können in beliebiger Reihenfolge durchgeführt werden. Das Verfahren kann ferner mehrere Formgebungsschritte und/oder mehrere Farbänderungsschritte umfassen.

In einer Ausführungsform werden dem Glas und der Glaskeramik vor oder nach der Bestrahlung oder vor oder nach der Wärmebehandlung die Form der gewünschten Dentalrestauration gegeben.

In einer weiteren Ausführungsform werden dem Glas und der Glaskeramik zwischen den für die Farbänderung erforderlichen Maßnahmen von Bestrahlung und Wärmebehandlung die Form der gewünschten Dentalrestauration gegeben.

Die Erfindung betrifft ferner eine mehrfarbige Dentalrestauration, die gemäß dem vorstehend beschriebenen erfindungsgemäßen Verfahren erhältlich ist.

Die nach dem erfindungsgemäßen Verfahren hergestellten mehrfarbigen Dentalrestaurationen zeichnen sich typischerweise dadurch aus, dass sie im Wesentlichen kontinuierliche Farbverläufe, das heißt Farbverläufe mit einer räumlichen Auflösung im Mikrometer- oder Nanometerbereich aufweisen. Mehrfarbige Dentalrestaurationen, die gemäß einem nach dem Stand der Technik üblichen Verfahren hergestellt wurden, weisen hingegen für gewöhnlich unterschiedlich gefärbte Farbschichten mit Schichtdicken im Millimeterbereich auf.

Die im erfindungsgemäßen Verfahren hergestellte mehrfarbige Dentalrestauration weist in der Regel vorteilhafte mechanische Eigenschaften auf. Ein hoher Gehalt an Lithiumdisilikat in der hergestellten Dentalrestauration ist aufgrund der überlegenen mechanischen Eigenschaften, wie beispielsweise der hohen Festigkeit, üblicherweise wünschenswert.

In einer bevorzugten Ausführungsform weist die nach dem erfindungsgemäßen Verfahren hergestellte Dentalrestauration eine biaxiale Festigkeit von mindestens 200 MPa, insbesondere mindestens 250 MPa, und/oder eine Bruchzähigkeit von mindestens 1,5 MPa m^{0,5} auf.

Bevorzugt weist die nach dem erfindungsgemäßen Verfahren hergestellte Dentalrestauration eine hohe chemische Beständigkeit mit einer Säurelöslichkeit gemäß ISO 6872 unterhalb von 100 µg/cm², insbesondere unterhalb von 50 µg/cm², auf.

Die Erfindung betrifft auch die Verwendung eines Glases oder einer Glaskeramik als Dentalmaterial, insbesondere zur Herstellung einer mehrfarbigen Dentalrestauration, wobei in mindestens einem Teil des Glases oder der Glaskeramik eine Farbänderung bewirkt wird, indem dieser Teil mit künstlicher elektromagnetischer Strahlung bestrahlt und dieser bestrahlte Teil einer Wärmebehandlung unterzogen wird.

Insbesondere betrifft die Erfindung auch die Verwendung eines Glases oder einer Glaskeramik als Dentalmaterial, bei der das Glas und die Glaskeramik einem der vorstehend beschriebenen Verfahren unterzogen werden. Alle Gläser und Glaskeramiken, die im Rahmen des erfindungsgemäßen Verfahrens beschrieben wurden, sind auch für die erfindungsgemäße Verwendung eines Glases oder einer Glaskeramik geeignet. Ebenso können alle Verfahrensschritte und Verfahrensparameter, die im Rahmen des erfindungsgemäßen Verfahrens beschrieben wurden, auch bei der erfindungsgemäßen Verwendung durchgeführt und gewählt werden.

Die Erfindung wird im Folgenden anhand von Ausführungsbeispielen näher erläutert.

### Beispiele

Es wurden Gläser mit den in den Tabellen 1 bis 5 angegebenen chemischen Zusammensetzungen hergestellt. Dazu wurde ein entsprechendes Gemenge an Rohstoffen, wie beispielsweise Oxiden, Carbonaten, Phosphaten und Halogeniden bei einer Schmelztemperatur (T_{S}) von 1000 bis 1650°C für eine Schmelzdauer (t_{S}) von 60 bis 300 min erschmolzen. Gegebenenfalls erfolgte die Herstellung der Glasschmelze in einem zweistufigen Verfahren mit zwei Schmelztemperaturen (T_{S1}, T_{S2}) und zwei Schmelzdauern (t_{S1}, t_{S2}).

Die Komponenten der Gläser und Glaskeramiken sind, sofern nicht anders angegeben, als Oxide berechnet, so wie es bei Gläsern und Glaskeramiken üblich ist.

Aus den Gläsern wurden mittels des erfindungsgemäßen Verfahrens mehrfarbige Dentalrestaurationen hergestellt, wobei die in den Tabellen 6 bis 16 angegebenen Bedingungen zur Bestrahlung und Wärmebehandlung verwendet wurden. In den Tabellen 6 bis 16 bedeuten
- T_{g}: Glasübergangstemperatur,
- T_{S}: Schmelztemperatur,
- t_{S}: Schmelzdauer,
- T_{N}: Keimbildungstemperatur,
- t_{N}: Keimbildungsdauer,
- QT: Quecksilberdampflampe, Hg-Hochdruckstrahler Typ TQ 150 von Heraeus, Hanau, Deutschland,
- LED: LED-Lichtquelle, Typ LCS-0310-03-23 von Mightex Systems, On-tario, Kanada, oder Typ M300L4 von ThorLabs Inc., NJ, USA,
- σ_{B}: Biaxialfestigkeit, bestimmt nach ISO 6872 (2008).

Die in den Tabellen 6 bis 16 aufgeführten Verfahrensschritte sind entsprechend ihrer zeitlichen Abfolge aufgeführt, wobei oben in der jeweiligen Tabelle aufgeführte Verfahrensschritte früher als weiter unten aufgeführte Verfahrensschritte erfolgen.

Für alle in den Beispielen genannten Wärmebehandlungen wurde ein Ofen des Typs Programat der Ivoclar Vivadent AG verwendet.

Kristallphasen von Glaskeramiken wurden mittels Röntgenbeugungsanalysen bestimmt.

Die Farbwerte (L*a*b) von hergestellten Glaskeramiken wurden im Messbereich von 400-700 nm mittels eines Spektrophotometers CM-3700d (Konica-Minolta) bestimmt. Der CR-Wert (Transluzenz) wurde gemäß British Standard BS 5612 bestimmt.

**Tabelle 1**

| **Beispiel** | **1-5** | **6-8** | **9** | **10** | **11** | **12-13** | **14-16** | **17** | **18** | **19** | **20** | **21** |
|---|---|---|---|---|---|---|---|---|---|---|---|---|
| *Zusammensetzung* | *Gew.-%* | *Gew.-%* | *Gew.-%* | *Gew.-%* | *Gew.-%* | *Gew.-%* | *Gew.-%* | *Gew.-%* | *Gew.-%* | *Gew.-%* | *Gew.-%* | *Gew.-%* |
| SiO₂ | 70,21 | 70,11 | 73,91 | 71,48 | 72,44 | 73,70 | 73,73 | 70,62 | 70,6 | 70,51 | 71,44 | 68,11 |
| Li₂O | 10,97 | 10,96 | 12,68 | 14,22 | 14,41 | 15,29 | 15,31 | 11,04 | 11,04 | 11,02 | 11,17 | 19,92 |
| Na₂O | 2,58 | 2,58 | 2,25 | 2,25 | 2,00 | | | 2,60 | 2,60 | 2,59 | 2,63 | |
| K₂O | 3,93 | 3,92 | 3,42 | 3,42 | 3,42 | 3,99 | 3,99 | 3,94 | 3,94 | 3,94 | 4,00 | 4,13 |
| Al₂O₃ | 6,80 | 6,79 | 3,70 | 4,60 | 3,70 | 3,51 | 3,51 | 6,83 | 6,83 | 6,82 | 6,92 | 3,64 |
| SnO* | 0,07 | 0,07 | 0,07 | 0,07 | 0,07 | | | | | | 0,07 | 0,08 |
| CeO₂ | 0,03 | 0,03 | 0,03 | 0,03 | 0,03 | 0,03 | 0,03 | 0,03 | 0,03 | 0,09 | 0,03 | 0,03 |
| Sb₂O₃ | 0,40 | 0,40 | 0,40 | 0,40 | 0,40 | | | | | | 0,41 | 0,44 |
| Ag₂O | 0,12 | 0,25 | 0,12 | 0,12 | 0,12 | | | | | 0,12 | 0,12 | 0,13 |
| AgCl* | | | | | | 0,09 | 0,03 | 0,03 | 0,05 | | | |
| P₂O₅ | 4,89 | 4,89 | 3,42 | 3,42 | 3,42 | 3,39 | 3,40 | 4,91 | 4,91 | 4,91 | 3,21 | 3,52 |

| | | | | | | | | | | | | |
|---|---|---|---|---|---|---|---|---|---|---|---|---|
| * als Rohstoff eingesetzt | | | | | | | | | | | | |

**Tabelle 2**

| **Beispiel** | **22** | **23 - 25** | **26** | **27** | **28** | **29** | **30** | **31** | **32** | **33** | **34 - 38** | **39** | **40 - 53** |
|---|---|---|---|---|---|---|---|---|---|---|---|---|---|
| *Zusammensetzung* | *Gew.-%* | *Gew.-%* | *Gew.-%* | *Gew.-%* | *Gew.-%* | *Gew.-%* | *Gew.-%* | *Gew.-%* | *Gew.-%* | *Gew.-%* | *Gew.-%* | *Gew.-%* | *Gew.-%* |
| SiO₂ | 73,67 | 75,17 | 72,78 | 74,55 | 73,11 | 72,89 | 72,89 | 73,68 | 73,71 | 73,716 | 73,713 | 72,771 | 73,527 |
| Li₂O | 13,09 | 13,35 | 13,92 | 12,36 | 14,54 | 14,50 | 14,50 | 15,30 | 15,31 | 15,31 | 15,31 | 15,11 | 15,27 |
| Na₂O | 2,23 | 2,25 | 2,24 | 2,21 | 1,75 | 2,00 | 2,00 | 3,99 | 3,99 | 3,99 | 3,99 | 3,94 | 3,98 |
| K₂O | | | | | 3,42 | 3,42 | 3,42 | | | | | | |
| Al₂O₃ | 7,33 | 5,55 | 7,38 | 7,28 | 3,70 | 3,70 | 3,70 | 3,51 | 3,51 | 3,51 | 3,51 | 3,46 | 3,50 |
| SnO* | 0,07 | 0,07 | 0,07 | 0,07 | | | | | 0,03 | 0,025 | 0,025 | 0,025 | 0,025 |
| CeO₂ | 0,03 | 0,03 | 0,03 | 0,03 | 0,031 | 0,031 | 0,031 | 0,06 | 0,03 | 0,032 | 0,032 | 0,031 | 0,032 |
| Sb₂O₃ | 0,40 | 0,40 | 0,40 | 0,40 | | | | | | | | | |
| Au₂O | | | | | | | | 0,07 | 0,02 | 0,004 | 0,007 | 0,0004 | 0,006 |
| Ag₂O | 0,12 | 0,12 | 0,10 | 0,06 | | | | | | | | | |
| AgCl* | | | | | 0,026 | | | | | 0,013 | 0,013 | 0,003 | 0,0105 |
| AgBr* | | | | | | 0,034 | | | | | | | |
| AgI* | | | | | | | 0,043 | | | | | | |
| P₂O₅ | 3,06 | 3,06 | 3,08 | 3,04 | 3,42 | 3,42 | 3,42 | 3,39 | 3,40 | 3,40 | 3,40 | 4,66 | 3,65 |

| | | | | | | | | | | | | | |
|---|---|---|---|---|---|---|---|---|---|---|---|---|---|
| * als Rohstoff eingesetzt | | | | | | | | | | | | | |

**Tabelle 3**

| **Beispiel** | **54** | **55** | **56** | **57** | **58** | **59** | **60** | **61** | **62** | **63** | **64** | **65** | **66** | **67** |
|---|---|---|---|---|---|---|---|---|---|---|---|---|---|---|
| *Zusammensetzung* | *Gew.-%* | *Gew.-%* | *Gew.-%* | *Gew.-%* | *Gew.-%* | *Gew.-%* | *Gew.-%* | *Gew.-%* | *Gew.-%* | *Gew.-%* | *Gew.-%* | *Gew.-%* | *Gew.-%* | *Gew.-%* |
| SiO₂ | 75,59 | 70,24 | 69,23 | 72,68 | 71,79 | 73,13 | 73,37 | 72,90 | 70,08 | 70,04 | 70,03 | 70,06 | 73,65 | 73,49 |
| Li₂O | 15,69 | 14,58 | 14,38 | 15,09 | 14,91 | 15,19 | 15,23 | 15,14 | 14,55 | 14,54 | 14,54 | 14,55 | 15,30 | 15,25 |
| K₂O | 4,08 | 3,80 | 3,75 | 3,93 | 3,89 | 3,96 | 3,97 | 3,95 | 3,79 | 3,79 | 3,78 | 3,79 | 3,99 | 3,98 |
| MgO | | | | | | | | | | | | 4,98 | | |
| CaO | | | | | | | | | 4,95 | | | | | |
| SrO | | | | | | | | | | 5,03 | | | | |
| ZnO | | | | | | | | | | | 5,01 | | | |
| Al₂O₃ | 3,60 | 3,33 | 3,29 | 3,46 | 3,42 | 3,37 | 3,50 | 3,47 | 3,34 | 3,33 | 3,34 | 3,34 | 3,51 | 3,50 |
| ZrO₂ | | | | | | | | 1,13 | | | | | | |
| SnO* | | | | | | | | | | | | | 0,03 | |
| CeO₂ | 0,03 | 0,03 | 0,03 | 1,00 | 0,03 | 0,03 | 0,03 | 0,03 | 0,03 | 0,03 | 0,03 | 0,03 | 0,06 | |
| CeF₃ | | | | | | | | | | | | | | 0,36 |
| AgCl* | 0,03 | 0,03 | 0,03 | 0,50 | 0,03 | 0,03 | 0,03 | 0,03 | 0,03 | 0,03 | 0,03 | 0,03 | 0,03 | 0,03 |
| CuCl* | | | | | | | | | | | | | 0,04 | |
| Ta₂O₅ | | | | | | 0,81 | | | | | | | | |
| Nb₂O₅ | | | | | | | 0,49 | | | | | | | |
| MoO₃ | | | | | 2,61 | | | | | | | | | |
| WO₃ | | | 6,10 | | | | | | | | | | | |
| P₂O₅ | 0,98 | 7,99 | 3,19 | 3,34 | 3,32 | 3,48 | 3,38 | 3,35 | 3,23 | 3,21 | 3,24 | 3,22 | 3,39 | 3,39 |

| | | | | | | | | | | | | | | |
|---|---|---|---|---|---|---|---|---|---|---|---|---|---|---|
| * als Rohstoff eingesetzt | | | | | | | | | | | | | | |

**Tabelle 4**

| **Beispiel** | **68** | **69** | **70** | **71** | **72** | **73** | **74 - 75** | **76** | **77** | **78 - 79** |
|---|---|---|---|---|---|---|---|---|---|---|
| *Zusammensetzung* | *Gew.-%* | *Gew.-%* | *Gew.-%* | *Gew.-%* | *Gew.-%* | *Gew.-%* | *Gew.-%* | *Gew.-%* | *Gew.-%* | *Gew.-%* |
| SiO₂ | 70,79 | 76,72 | 68,86 | 73,39 | 68,63 | 79,96 | 71,29 | 73,03 | 72,79 | 72,10 |
| Li₂O | 7,63 | 8,27 | 14,39 | 15,18 | 15,71 | 15,29 | 12,40 | 10,38 | 10,34 | 15,02 |
| Na₂O | | | | | | 0,94 | | | | |
| K₂O | 9,57 | 3,61 | 9,99 | 3,96 | 3,93 | 1,07 | 3,40 | 3,64 | 3,63 | 3,95 |
| MgO | 1,71 | 1,85 | | | | | 1,60 | 1,86 | 1,85 | |
| CaO | 3,10 | 3,36 | | | | | 2,32 | 3,38 | 3,37 | |
| Al₂O₃ | 3,57 | 3,86 | 3,40 | 3,35 | 3,33 | | 4,02 | 3,89 | 3,87 | 3,34 |
| Er₂O₃ | | | | 0,70 | | | | | 0,34 | |
| SnO₂ | 0,03 | 0,03 | | | | | 0,07 | 0,03 | 0,03 | |
| GeO₂ | | | | | 4,74 | | | | | 1,91 |
| CeO₂ | 0,23 | 0,24 | 0,03 | 0,03 | 0,03 | 0,03 | 0,03 | 0,12 | 0,12 | 0,03 |
| Sb₂O₃ | | | | | | | 0,42 | | | |
| Ag₂O | | | | | | | 0,12 | | | |
| AgCl* | 0,05 | 0,05 | 0,03 | 0,03 | 0,03 | 0,03 | | 0,05 | 0,05 | 0,03 |
| P₂O₅ | 3,32 | 2,01 | 3,30 | 3,36 | 3,60 | 2,68 | 4,33 | 3,62 | 3,61 | 3,62 |

| | | | | | | | | | | |
|---|---|---|---|---|---|---|---|---|---|---|
| * als Rohstoff eingesetzt | | | | | | | | | | |

**Tabelle 5**

| **Beispiel** | **80** | **81** | **82** | **83** | **84** | **85** | **86** | **87** | **88** | **89** | **90** |
|---|---|---|---|---|---|---|---|---|---|---|---|
| *Zusammensetzung* | *Gew.-%* | *Gew.-%* | *Gew.-%* | *Gew.-%* | *Gew.-%* | *Gew.-%* | *Gew.-%* | *Gew.-%* | *Gew.-%* | *Gew.-%* | *Gew.-%* |
| SiO₂ | 73,85 | 75,59 | 75,50 | 76,33 | 77,47 | 76,89 | 73,08 | 78,50 | 69,11 | 78,08 | 74,65 |
| Li₂O | 7,96 | 8,15 | 8,14 | 8,23 | 6,42 | 8,29 | 7,88 | 8,46 | 7,45 | 8,42 | 8,05 |
| K₂O | 5,67 | 3,57 | 3,56 | 3,60 | 3,51 | 3,63 | 3,45 | 3,71 | 3,26 | 3,69 | 3,53 |
| MgO | 1,78 | 1,82 | 1,82 | 1,84 | 1,79 | | 4,96 | 1,90 | 1,66 | 1,88 | 1,80 |
| CaO | 3,23 | 3,31 | 3,31 | 3,35 | 3,26 | 3,37 | 3,21 | 3,43 | 3,03 | | 4,38 |
| Al₂O₃ | 3,72 | 3,81 | 3,80 | 3,85 | 3,74 | 3,88 | 3,68 | | 11,93 | 3,94 | 3,77 |
| SnO₂ | 0,03 | 0,03 | 0,03 | 0,03 | 0,03 | 0,03 | 0,03 | 0,03 | 0,03 | 0,03 | 0,03 |
| CeO₂ | 0,24 | 0,12 | 0,24 | 0,24 | 0,24 | 0,24 | 0,24 | 0,24 | 0,23 | 0,24 | 0,24 |
| AgCl* | 0,05 | 0,05 | 0,05 | 0,05 | 0,05 | 0,05 | 0,05 | 0,05 | 0,05 | 0,05 | 0,05 |
| P₂O₅ | 3,47 | 3,55 | 3,55 | 2,48 | 3,49 | 3,62 | 3,42 | 3,68 | 3,25 | 3,67 | 3,50 |

| | | | | | | | | | | | |
|---|---|---|---|---|---|---|---|---|---|---|---|
| * als Rohstoff eingesetzt | | | | | | | | | | | |

### Beispiele 1 bis 8: Farbänderung durch Bestrahlung eines Glases mit einer Quecksilberdampflampe und Wärmebehandlung

An den Lithiumsilikatgläsern der Beispiele 1 bis 5 wurde zunächst eine Wärmebehandlung zur Bildung von Kristallisationskeimen durchgeführt. Anschließend wurden die keimhaltigen Gläser für 15 bis 60 min einer Bestrahlung mit einer Quecksilberdampflampe (Hg-Hochdruckstrahler Typ TQ 150, Heraeus, Hanau, Deutschland) und für 15 bis 60 min einer Wärmebehandlung bei 470 bis 610°C unterzogen. Die jeweils genutzten Bedingungen, die durch die Bestrahlung und Wärmebehandlung bewirkten Farben sowie die bestimmten Kristallphasen der hergestellten Glaskeramik sind in der Tabelle 6 angegeben. Eine Keimbildungsdauer von 0 min bedeutet, dass die Gläser nach dem Gießen in einen auf die Keimbildungstemperatur eingestellten Ofen überführt und darin ohne Haltezeit abgekühlt wurden.

Nach Bestrahlung der keimhaltigen Gläser und anschließende Wärmebehandlung konnten in den Glaskeramiken Gelb- und Rotfärbungen festgestellt werden. Es wurde ferner z.B. bei den Glaskeramiken der Beispiele 1 und 2 beobachtet, dass eine längere Bestrahlung eine intensivere und dunklere Färbung bewirkte.

Aus dem Vergleich der Beispiele 3 bis 5 lässt sich zudem erkennen, dass die bei der Farbänderung bewirkte Farbe von der Temperatur der Wärmebehandlung abhängig war. Die Wärmebehandlung von Beispiel 4, die verglichen mit den Beispielen 3 und 5 bei einer hohen Temperatur erfolgte, resultierte in einer intensiveren und dunkleren Färbung der Glaskeramik als in den Glaskeramiken der Beispiele 3 und 5.

In der Glaskeramik von Beispiel 4 wurde nach Bestrahlung und Wärmebehandlung eine verringerte Transluzenz beobachtet.

Aus dem Vergleich der Farbe der bestrahlten und wärmebehandelten Bereiche der Glaskeramiken von den Beispielen 6 und 8 ist ersichtlich, dass durch eine längere Bestrahlung eine intensivere und dunklere Farbe bewirkt werden konnte.

Die in den Beispielen 1 bis 5 hergestellten Glaskeramiken enthielten 0,12 Gew.-% Ag₂O, wohingegen 0,25 Gew.-% Ag₂O in den Glaskeramiken der Beispiele 6 bis 8 enthalten war. Aus den Farben, die in den Glaskeramiken der Beispiele 2 und 6 erzeugt wurden, wird deutlich, dass eine intensivere Farbe bewirkt werden kann, wenn das Glas oder die Glaskeramik eine größere Menge Ag enthält.

An dem Glas und der Glaskeramik von Beispiel 7 wurde die Farbänderungen in zwei Schritten, die jeweils eine Bestrahlung und eine Wärmebehandlung umfassten, bewirkt. Die erste Farbänderung umfasste eine Bestrahlung mit einer Quecksilberdampflampe von keimhaltigem Glas und eine Wärmebehandlung. Die Wärmebehandlung der ersten Farbänderung bewirkte auch eine Kristallisation des keimhaltigen Glases. Die dabei entstandene Glaskeramik wurde mit einer LED-Lichtquelle (LCS-0310-03-23 von Mightex Systems, ON, Kanada) erneut bestrahlt und einer weiteren Wärmebehandlung zur Farbänderung und weiteren Kristallisation unterzogen.

**Tabelle 6**

| **Beispiel** | **1** | **2** | **3** | **4** | **5** | **6** | **7** | **8** |
|---|---|---|---|---|---|---|---|---|
| T_{g} /°C | | | | | | | | |
| T_{S} /°C | 1500 | 1500 | 1500 | 1500 | 1500 | 1500 | 1500 | 1500 |
| t_{S} /min | 120 | 120 | 120 | 120 | 120 | 120 | 120 | 120 |
| T_{N} /°C | 530 | 530 | 530 | 530 | 530 | 530 | 530 | 530 |
| t_{N} /min | 0 | 0 | 0 | 0 | 0 | 0 | 0 | 0 |
| **Bestrahlung Glas** | | | | | | | | |
| Strahlungsquelle Wellenlänge /nm | QT | QT | QT | QT | QT | QT | QT | QT |
| Dauer /min Intensität (%) | 15 | 60 | 15 | 15 | 15 | 60 | 15 | 45 |
| **1 .Wärmebehandlung** | | | | | | | | |
| Temperatur /°C | 550 | 550 | 550 | 610 | 470 | 550 | 550 | 550 |
| Dauer /min | 60 | 60 | 15 | 15 | 15 | 60 | 15 | 60 |
| **Bestrahlung Glaskeramik** | | | | | | | | |
| Strahlungsquelle | | | | | | | LED | |
| Wellenlänge (nm) | | | | | | | 310 | |
| Dauer (min) | | | | | | | 15 | |
| Intensität (%) | | | | | | | 100 | |
| **2 .Wärmebehandlung** | | | | | | | | |
| Temperatur (°C) | | | | | | | 600 | |
| Dauer (min) | | | | | | | 10 | |
| **Kristallphasen** | Li₂SiO₃ | Li₂SiO₃ | Li₂SiO₃ | Li₂SiO₃ | Li₂SiO₃ | Li₂SiO₃ | Li₂SiO₃ | Li₂SiO₃ |
| | Li₃PO₄ | Li₃PO₄ | Li₃PO₄ | Li₃PO₄ | Li₃PO₄ | Li₃PO₄ | Li₃PO₄ | Li₃PO₄ |
| **Farbeindruck der Glaskeramik** | gelbrot | orangerot | gelb | orange | grüngrau | rotschwarz | orangerosa | dunkelrotbraun |

### Beispiele 9 bis 12: Farbänderung durch Bestrahlung eines Glases mit einer LED mit 300 nm Wellenlänge und Wärmebehandlung

Die Lithiumsilikatgläser der Beispiele 9 bis 12 wurden für 15 Minuten mittels einer LED (M300L4 von ThorLabs Inc., NJ, USA) mit Strahlung einer Wellenlänge von 300 nm bestrahlt.

Die bestrahlten Gläser wurden den in Tabelle 7 angegebenen Wärmebehandlungen unterzogen. Die durch die Farbänderung in der hergestellten Glaskeramik bewirkten Farben sind ebenfalls in der Tabelle 7 angegeben. Es konnten in den Beispielen 9 bis 11 in Glaskeramiken mit CeO₂, Ag, Sb₂O₃ und SnO unterschiedliche Färbungen festgestellt werden. Im Beispiel 12, in der das bestrahlte Glas Ce, Ag und Cl enthielt, wurde eine Braun-Gelbfärbung beobachtet.

### Beispiele 13 bis 18: Farbänderung in einer Ce-, Ag- und Clhaltigen Glaskeramik durch Bestrahlung mit einer LED und Wärmebehandlung

Die Lithiumsilikatgläser der Beispiele 13 bis 18 wurden zunächst einer Wärmebehandlung zur Keimbildung und einer Wärmebehandlung zur Kristallisation unterzogen. Die Glaskeramiken wurden für 15 min mit einer LED (LCS-0310-03-23 von Mightex Systems, ON, Kanada) bestrahlt, wobei die Strahlung einen Anteil mit einer Wellenlänge von 310 nm enthielt. Anschließend wurden die bestrahlten Glaskeramiken einer weiteren Wärmebehandlung unterzogen. Die durch die Farbänderung bewirkten Farben sind ebenfalls in der Tabelle 7 angegeben.

Für die in Beispiel 16 hergestellte Glaskeramik wurde eine sehr hohe und für den Einsatz als Dentalmaterial vorteilhafte biaxiale Festigkeit von 604 ± 104 MPa bestimmt.

### Beispiele 19 bis 21: Farbänderung in einer Glaskeramik durch Bestrahlung mit einer LED oder Quecksilberdampflampe und Wärmebehandlung

Die Lithiumsilikatgläser der Beispiele 19 bis 21 wurden zunächst einer Wärmebehandlung zur Keimbildung und einer Wärmebehandlung zur Kristallisation unterzogen. Die Glaskeramiken wurden mit einer LED (LCS-0310-03-23 von Mightex Systems, ON, Kanada) oder Quecksilberdampflampe für 15 min bestrahlt und einer weiteren Wärmebehandlung unterzogen. Die durch die Farbänderung bewirkten Farben sind in der Tabelle 8 angegeben.

### Beispiele 22 bis 27: Farbänderung in einer Glaskeramik, die mittels zwei Wärmebehandlungen kristallisiert wurde, durch Bestrahlung mit einer LED und Wärmebehandlung

Die Lithiumsilikatgläser der Beispiele 22 bis 27 wurden zunächst einer Wärmebehandlung zur Keimbildung und zwei Wärmebehandlungen zur Kristallisation unterzogen. Die Glaskeramiken wurden mit einer LED (LCS-0310-03-23 von Mightex Systems, ON, Kanada) für 15 min bestrahlt und einer weiteren Wärmebehandlung unterzogen.

Bei den Glaskeramiken der Beispiele 23 und 24 konnte durch eine längere Dauer der Wärmebehandlung nach der Bestrahlung eine dunklere Farbe bewirkt werden.

Bei den Glaskeramiken der Beispiele 23 und 25 konnte durch eine höhere Temperatur der Wärmebehandlung nach der Bestrahlung eine dunklere und intensivere Farbe bewirkt werden.

**Tabelle 7**

| **Beispiel** | **9** | **10** | **11** | **12** | **13** | **14** | **15** | **16** | **17** | **18** |
|---|---|---|---|---|---|---|---|---|---|---|
| T_{g} /°C | 458,2 | 451,7 | 449,9 | | 453,6 | 453,6 | 453,6 | 453,6 | | |
| T_{S} /°C | | | | 1400 | 1400 | 1400 | 1400 | 1400 | 1400 | 1400 |
| t_{S} /min | | | | 60 | 60 | 60 | 60 | 60 | 60 | 60 |
| T_{N} /°C | | | | 490 | 490 | 490 | 490 | 490 | 530 | 530 |
| t_{N} /min | | | | 0 | 0 | 10 | 10 | 10 | 0 | 0 |
| **Bestrahlung Glas** | | | | | | | | | | |
| Strahlungsquelle | LED | LED | LED | LED | | | | | | |
| Wellenlänge /nm | 300 | 300 | 300 | 300 | | | | | | |
| Dauer /min | 15 | 15 | 15 | 15 | | | | | | |
| Intensität /% | 100 | 100 | 100 | 100 | | | | | | |
| **1 .Wärmebehandlung** | | | | | | | | | | |
| Temperatur /°C | 550 | 550 | 550 | 550 | 850 | 850 | 850 | 900 | 800 | 600 |
| Dauer /min | 15 | 15 | 15 | 15 | 15 | 15 | 15 | 60 | 10 | 10 |
| **Bestrahlung Glaskeramik** | | | | | | | | | | |
| Strahlungsquelle | | | | | LED | LED | LED | LED | LED | LED |
| Wellenlänge /nm | | | | | 310 | 310 | 310 | 310 | 310 | 310 |
| Dauer /min | | | | | 15 | 15 | 15 | 15 | 15 | 15 |
| Intensität /mA | | | | | 400 | 400 | 400 | 400 | 400 | 400 |
| **2 .Wärmebehandlung** | | | | | | | | | | |
| Temperatur /°C | 650 | 650 | 650 | | 550 | 550 | 650 | 550 | 550 | 550 |
| Dauer /min | 20 | 20 | 20 | | 30 | 30 | 30 | 15 | 30 | 30 |
| **3. Wärmebehandlung** | | | | | | | | | | |
| Temperatur /°C | | | 800 | | | | | | | |
| Dauer /min | | | 10 | | | | | | | |
| **Kristallphasen** | Li₂SiO₃ | Li₂Si₂O₅ | Li₂Si₂O₅ | Li₂Si₂O₅ | Li₂Si₂O₅ | | | Li₂Si₂O₅ | Li₂Si₂O₅ | Li₂SiO₃ |
| | Li₃PO₄ | Li₂SiO₃ | Li₂SiO₃ | Li₂SiO₃ | Li₂SiO₃ | | | Li₃PO₄ | Li₂SiO₃ | Li₃PO₄ |
| | | Li₃PO₄ | Li₃PO₄ | Li₃PO₄ | Li₃PO₄ | | | | Li₃PO₄ | |
| σ_{B} /MPa | | | | | | | | 604 ± 104 | | |
| CR (Transluzenz) | | | | | | | | 88,3 | | |
| **Farbeindruck der Glaskeramik** | leicht gelb | intensiv gelb | gelb | braungelb | gelb | gelb | gelbbraun | gelbleicht braun | gelb, leicht braun | gelb, leicht braun |

**Tabelle 8**

| **Beispiel** | **19** | **20** | **21** | **22** | **23** | **24** | **25** | **26** | **27** |
|---|---|---|---|---|---|---|---|---|---|
| T_{g} /°C | | | 441,8 | | | | | | |
| T_{S} /°C | 1400 | 1500 | 1400 | | | | | | |
| t_{S} /min | 60 | 120 | 60 | | | | | | |
| T_{N} /°C | 530 | 530 | 460 | 480 | 480 | 480 | 480 | 470 | 510 |
| t_{N} /min | 0 | 0 | 10 | 10 | 10 | 10 | 10 | 10 | 10 |
| **1 .Wärmebehandlung** | | | | | | | | | |
| Temperatur /°C | 600 | 750 | 600 | 600 | 590 | 590 | 590 | 600 | 600 |
| Dauer /min | 10 | 10 | 10 | 20 | 40 | 40 | 40 | 20 | 20 |
| **Bestrahlung Glaskeramik** | | | | | | | | | |
| Strahlungsquelle | LED | QT | LED | | | | | | |
| Wellenlänge /nm | 310 | | 310 | | | | | | |
| Dauer /min | 15 | 60 | 15 | | | | | | |
| Intensität /mA | 400 | | 400 | | | | | | |
| **2 .Wärmebehandlung** | | | | | | | | | |
| Temperatur /°C | 550 | 550 | 550 | 800 | 780 | 780 | 780 | 800 | 800 |
| Dauer /min | 60 | 30 | 30 | 10 | 15 | 15 | 15 | 10 | 10 |
| **Bestrahlung Glaskeramik** | | | | | | | | | |
| Strahlungsquelle | | | | LED | LED | LED | LED | LED | LED |
| Wellenlänge /nm | | | | 310 | 310 | 310 | 310 | 310 | 310 |
| Dauer /min | | | | 15 | 15 | 15 | 15 | 15 | 15 |
| Intensität /mA | | | | 400 | 400 | 400 | 400 | 400 | 400 |
| **3. Wärmebehandlung** | | | | | | | | | |
| Temperatur /°C | | | | 650 | 700 | 700 | 580 | | 650 |
| Dauer /min | | | | 5 | 15 | 30 | 15 | | 15 |
| **Kristallphasen** | | | | Li₂Si₂O₅ | Li₂Si₂O₅ | Li₂Si₂O₅ | Li₂Si₂O₅ | Li₂Si₂O₅ | Li₂Si₂O₅ |
| | | | | Li_{0,155}Al_{0,155}- | Cristobalit | Cristobalit | Cristobalit | Li₃PO₄ | Li₃PO₄ |
| | | | Li₂SiO₃ | Si_{0,845}O₂ | | | | LiₓAlₓSi₃₋ₓO₆ | β-Quarz |
| | | | Li₃PO₄ | Li₃PO₄ | Li₃PO₄ | Li₃PO₄ | Li₃PO₄ | (Virgilit) | Cristobalit |
| | | | | LiAlSi₂O₆ | LiAlSi₂O₆ | LiAlSi₂O₆ | LiAlSi₂O₆ | Li_{0,4}Al_{0,4}-Si_{0,6}O₂ | |
| **Farbeindruck der Glaskeramik** | gelbleicht braun | orangebraun | gelbleicht braun | gelb | intensiv gelb | orangebraun | leicht gelb | gelb | gelb |

### Beispiele 28 bis 30: Farbänderung in einer Glaskeramik, die Ag sowie Cl oder Br oder I enthält, durch Bestrahlung mit einer LED und Wärmebehandlung

Es wurden Gläser unter Verwendung von AgCl, AgBr oder AgI als Rohstoff hergestellt und einer Wärmebehandlung zur Keimbildung sowie zwei Wärmebehandlungen zur Kristallisation unterzogen. Die Glaskeramiken wurden bestrahlt und einer erneuten Wärmebehandlung unterzogen.

**Tabelle 9**

| **Beispiel** | **28** | **29** | **30** |
|---|---|---|---|
| T_{N} /°C | 470 | 460 | 460 |
| t_{N} /min | 10 | 30 | 30 |
| **1.Wärmebehandlung** | | | |
| Temperatur /°C | 630 | 600 | 700 |
| Dauer /min | 15 | 30 | 10 |
| **2.Wärmebehandlung** | | | |
| Temperatur /°C | 880 | 830 | 870 |
| Dauer /min | 1 | 5 | 2 |
| **Bestrahlung Glaskeramik** | | | |
| Strahlungsquelle | LED | LED | LED |
| Wellenlänge /nm | 310 | 310 | 310 |
| Dauer /min | 15 | 15 | 15 |
| Intensität /mA | 400 | 400 | 400 |
| **3.Wärmebehandlung** | | | |
| Temperatur /°C | 650 | 650 | 550 |
| Dauer /min | 15 | 15 | 60 |
| **Kristallphasen** | Li₂Si₂O₅ | Li₂Si₂O₅ | Li₂Si₂O₅ |
| | | | Li₂SiO₃ |
| | Li₃PO₄ | Li₃PO₄ | Li₃PO₄ |
| **σ_{B} /MPa** | | 554 | 510 |
| **Farbeindruck der Glaskeramik** | gelb | gelb | gelb |

Nach Bestrahlung und Wärmebehandlung wurde in Glaskeramiken der Beispiele 28 bis 30 eine Gelbfärbung beobachtet.

### Beispiele 31 bis 53: Farbänderung in einer Glaskeramik, die Ce sowie Au und gegebenenfalls Ag enthält

Die Zusammensetzungen der Beispiele 31 und 32 enthielten Ce und Au. In denen der Beispiele 33 bis 53 war neben Ce und Au auch Ag enthalten.

Es wurden Lithiumsilikatgläser in einem zweistufigen Schmelzverfahren hergestellt, wobei der erste Schritt für 30 min bei 1000°C und der zweite Schritt für 60 min bei 1450°C erfolgte. Daraufhin wurden die Lithiumsilikatgläser einer Wärmebehandlung zur Keimbildung für 10 min bei 480°C unterzogen.

Nach einer ersten Wärmebehandlung zur Kristallisation wurden die Beispiele 31 bis 39 einer Bestrahlung mit einer LED-Lichtquelle (LCS-0310-03-23 von Mightex Systems, ON, Kanada) bei 310 nm, 400 mA unterzogen. Die bestrahlten Glaskeramiken wurden einer weiteren Wärmebehandlung unterzogen und der Farbeindruck wurde bestimmt.

### Beispiele 31 und 32

Diese Beispiele veranschaulichen, dass durch Bestrahlung und Wärmebehandlung einer Glaskeramik, die Ce und Au enthält, eine rötliche Färbung erzielt werden kann. Durch einen höheren Gehalt an Au konnte eine intensivere Rotfärbung erzielt werden.

### Beispiele 33 bis 35

Diese Beispiele veranschaulichen, dass durch eine längere Wärmebehandlung nach der Bestrahlung einer Glaskeramik mit Ce, Au und Ag eine intensivere Färbung erzielt werden kann. Es konnte insbesondere beobachtet werden, dass eine längere Wärmebehandlung eine stärkere Rotfärbung bewirkt.

### Beispiele 36 bis 38

In diesen Beispielen konnte beobachtet werden, dass eine höhere Temperatur der auf die Bestrahlung folgenden Wärmebehandlung eine intensivere Färbung bewirkt. Insbesondere wurde bei einer höheren Temperatur eine stärkere Rotfärbung festgestellt.

Die Beispiele 40 bis 53 wurden zunächst zwei Wärmebehandlungen zur Kristallisation unterzogen und dann mit einer LED-Lichtquelle (LCS-0310-03-23 von Mightex Systems, ON, Kanada) bei 310 nm, 400 mA bestrahlt. Die bestrahlten Glaskeramiken wurden einer weiteren Wärmebehandlung unterzogen und es wurde der Farbeindruck bestimmt.

### Beispiele 40 bis 42

Diese Beispiele veranschaulichen, dass durch eine längere Wärmebehandlung nach der Bestrahlung einer Glaskeramik mit Ce, Au und Ag eine intensivere Färbung, insbesondere eine stärkere Rotfärbung erzielt werden kann.

### Beispiele 42 bis 44

In diesen Beispielen konnte beobachtet werden, dass eine höhere Temperatur der auf die Bestrahlung folgenden Wärmebehandlung eine intensivere Färbung in Glaskeramiken mit Ce, Au und Ag bewirkt. Insbesondere wurde bei einer höheren Temperatur eine stärkere Rotfärbung festgestellt.

### Beispiele 45 bis 47

Der Vergleich dieser Beispiele mit den Beispielen 42 bis 44 zeigt, dass eine gewünschte Farbwirkung erreicht werden kann, wenn die Wärmebehandlung der bestrahlten Glaskeramik für eine kürzere Dauer aber bei einer erhöhten Temperatur erfolgt. Erneut konnte eine intensivere Färbung, insbesondere eine stärkere Rotfärbung, durch eine höhere Temperatur bewirkt werden.

### Beispiele 48 bis 50 und Beispiele 51 bis 53

Diese Beispiele veranschaulichen, dass durch eine längere Bestrahlungsdauer eine intensivere Färbung bewirkt werden kann. Insbesondere wurde bei einer längeren Bestrahlungsdauer eine stärkere Rotfärbung beobachtet.

**Tabelle 10**

| **Beispiel** | **31** | **32** | **33** | **34** | **35** | **36** | **37** | **38** | **39** |
|---|---|---|---|---|---|---|---|---|---|
| **1 .Wärmebehandlung** | | | | | | | | | |
| Temperatur /°C | 900 | 900 | 900 | 900 | 900 | 900 | 900 | 900 | 820 |
| Dauer /min | 60 | 60 | 60 | 60 | 60 | 60 | 60 | 60 | 10 |
| **Bestrahlung Glaskeramik** | | | | | | | | | |
| Strahlungsquelle | LED | LED | LED | LED | LED | LED | LED | LED | LED |
| Wellenlänge /nm | 310 | 310 | 310 | 310 | 310 | 310 | 310 | 310 | 310 |
| Dauer /min | 15 | 15 | 15 | 60 | 15 | 15 | 15 | 15 | 15 |
| **2 .Wärmebehandlung** | | | | | | | | | |
| Temperatur /°C | 600 | 600 | 600 | 600 | 600 | 650 | 600 | 550 | 600 |
| Dauer /min | 15 | 15 | 15 | 20 | 30 | 10 | 10 | 10 | 10 |
| **Kristallphasen** | Li₂Si₂O₅ | Li₂Si₂O₅ | | | | | | | |
| | Li₃PO₄ | Li₃PO₄ | | | | | | | |
| **Farbeindruck der Glaskeramik** | rotviolett | rosa | gelbleicht orange | gelbleicht rot | gelb-rot | rot | gelb-rot | gelb | leicht gelb |

**Tabelle 11**

| **Beispiel** | **40** | **41** | **42** | **43** | **44** | **45** | **46** | **47** | **48** |
|---|---|---|---|---|---|---|---|---|---|
| **Bestrahlung Glaskeramik** | | | | | | | | | |
| Strahlungsquelle | LED | LED | LED | LED | LED | LED | LED | LED | LED |
| Wellenlänge /nm | 310 | 310 | 310 | 310 | 310 | 310 | 310 | 310 | 310 |
| Dauer /min | 15 | 15 | 15 | 15 | 15 | 15 | 15 | 15 | 15 |
| **3. Wärmebehandlung** | | | | | | | | | |
| Temperatur /°C | 650 | 650 | 650 | 700 | 600 | 850 | 800 | 750 | 800 |
| Dauer /min | 45 | 30 | 15 | 15 | 15 | 1 | 1 | 1 | 1 |
| **Kristallphasen** | Li₂Si₂O₅ | Li₂Si₂O₅ | Li₂Si₂O₅ | Li₂Si₂O₅ | Li₂Si₂O₅ | Li₂Si₂O₅ | Li₂Si₂O₅ | Li₂Si₂O₅ | Li₂Si₂O₅ |
| | Li₃PO₄ | Li₃PO₄ | Li₃PO₄ | Li₃PO₄ | Li₃PO₄ | Li₃PO₄ | Li₃PO₄ | Li₃PO₄ | Li₃PO₄ |
| **Farbeindruck der Glaskeramik** | rot-gelb | gelbleicht rot | gelbsehr leicht rot | rot-gelb | leicht gelb | rosa | orangerosa | gelb | orangerosa |

**Tabelle 12**

| **Beispiel** | **49** | **50** | **51** | **52** | **53** |
|---|---|---|---|---|---|
| **Bestrahlung Glaskeramik** | | | | | |
| Strahlungsquelle | LED | LED | LED | LED | LED |
| Wellenlänge /nm | 310 | 310 | 310 | 310 | 310 |
| Dauer /min | 10 | 5 | 15 | 10 | 5 |
| **3. Wärmebehandlung** | | | | | |
| Temperatur /°C | 800 | 800 | 770 | 770 | 770 |
| Dauer /min | 1 | 1 | 1 | 1 | 1 |
| **Kristallphasen** | Li₂Si₂O₅ | Li₂Si₂O₅ | Li₂Si₂O₅ | Li₂Si₂O₅ | Li₂Si₂O₅ |
| | Li₃PO₄ | Li₃PO₄ | Li₃PO₄ | Li₃PO₄ | Li₃PO₄ |
| **Farbeindruck der Glaskeramik** | leicht orangerosa | sehr leicht orangerosa | leicht orange | gelborange | gelbleicht orange |

### Beispiele 54 bis 74 und 76 bis 78: Farbänderung in einer Glaskeramik durch Bestrahlung mit einer LED und Wärmebehandlung

Die Gläser dieser Beispiele wurden zunächst einer Wärmebehandlung zur Keimbildung und einer Wärmebehandlungen zur Kristallisation unterzogen. Die Glaskeramiken wurden mit einer LED-Lichtquelle (LCS-0310-03-23 von Mightex Systems, ON, Kanada) bestrahlt und einer weiteren Wärmebehandlung unterzogen.

Diese Beispiele veranschaulichen, dass eine Farbänderung mittels Bestrahlung und Wärmebehandlung in Glaskeramiken mit deutlich unterschiedlichen Zusammensetzungen, z.B. mit geringem (Beispiel 54) oder hohem Gehalt an P₂O₅ (Beispiel 55), hohem Gehalt an Ce und Ag (Beispiel 57) oder mit Gehalt an ZrO₂ (Beispiel 61), erfolgen kann.

### Beispiele 71 und 77

Diese Beispiele veranschaulichen, dass die durch Bestrahlung und Wärmebehandlung bewirkte Farbänderung mit anderen Färbeverfahren kombiniert werden kann. Das in den Zusammensetzungen der Beispiele 71 und 77 enthaltene Er₂O₃ führte in der hergestellten Glaskeramik zu einer leichten Rotfärbung, wenngleich der Gesamteindruck der erfindungsgemäß bestrahlten und wärmebehandelten Glaskeramik gelb war. Die Rotfärbung der Glaskeramiken der Beispiele 71 und 77 wurde durch eine Bestimmung der a*-Werte quantifiziert. Die a*-Werte waren verglichen mit Glaskeramiken, die keine rotfärbende Komponente enthielten, wie Beispiel 70, erhöht.

### Beispiel 78

Die Untersuchung der chemischen Beständigkeit nach ISO 6872 (2008) der Glaskeramik gemäß Beispiel 78 ergab eine Säurelöslichkeit von 18 µg/cm².

### Beispiele 80 bis 90: Farbänderung in Tiefquarz- und Lithiumalumosilikat-Glaskeramiken

Die Gläser wurden in einem zweistufigen Schmelzverfahren hergestellt und einer Wärmebehandlung zur Keimbildung unterzogen. Nach einer Wärmebehandlung zur Kristallisation wurde eine Farbänderung durch Bestrahlung und Wärmebehandlung durchgeführt und die erhaltenen Kristallphasen wurden bestimmt.

Die Beispiele veranschaulichen, dass eine Farbänderung durch Bestrahlung und Wärmebehandlung auch in Glaskeramiken, die Tiefquarz oder Lithiumalumosilikat als Hauptkristallphase aufweisen, bewirkt werden kann.

**Tabelle 13**

| **Beispiel** | **54** | **55** | **56** | **57** | **58** | **59** | **60** | **61** | **62** | **63** | **64** | **65** |
|---|---|---|---|---|---|---|---|---|---|---|---|---|
| Tg /°C | 455,5 | 474,7 | 461,9 | | | | | | 453,3 | 451,7 | 450,4 | 452,2 |
| T_{S1} /°C | 1400 | 1400 | 1400 | 1450 | 1400 | 1400 | 1400 | 1400 | 1400 | 1400 | 1400 | 1400 |
| t_{S1} /min | 60 | 60 | 60 | 30 | 60 | 60 | 60 | 60 | 60 | 60 | 60 | 60 |
| T_{S2} /°C | | | | 1650 | | | | | | | | |
| t_{S2} /min | | | | 30 | | | | | | | | |
| T_{N} /°C | 490 | 490 | 540 | 480 | 480 | 490 | 500 | 500 | 480 | 470 | 470 | 470 |
| t_{N} /min | 10 | 10 | 10 | 10 | 10 | 10 | 10 | 10 | 10 | 10 | 10 | 10 |
| **1 .Wärmebehandlung** | | | | | | | | | | | | |
| Temperatur /°C | 840 | 820 | 820 | 900 | 810 | 850 | 800 | 840 | 700 | 830 | 810 | 780 |
| Dauer /min | 15 | 15 | 30 | 60 | 10 | 10 | 10 | 10 | 30 | 30 | 30 | 30 |
| **Bestrahlung Glaskeramik** | | | | | | | | | | | | |
| Strahlungsquelle | LED | LED | LED | LED | LED | LED | LED | LED | LED | LED | LED | LED |
| Wellenlänge /nm | 310 | 310 | 310 | 310 | 310 | 310 | 310 | 310 | 310 | 310 | 310 | 310 |
| Dauer /min | 15 | 15 | 15 | 15 | 15 | 15 | 15 | 15 | 15 | 15 | 15 | 15 |
| Intensität /mA | 400 | 400 | 400 | 400 | 400 | 400 | 400 | 400 | 400 | 400 | 400 | 400 |
| **2 .Wärmebehandlung** | | | | | | | | | | | | |
| Temperatur /°C | 550 | 550 | 550 | 550 | 550 | 550 | 550 | 550 | 550 | 550 | 550 | 600 |
| Dauer /min | 15 | 15 | 15 | 15 | 15 | 60 | 60 | 15 | 60 | 60 | 60 | 60 |
| **3. Wärmebehandlung** | | | | | | | | | | | | |
| Temperatur /°C | | | | 550 | | | | | | | | |
| Dauer /min | | | | 45 | | | | | | | | |
| **Kristallphasen** | | Li₂Si₂O₅ | Li₂Si₂O₅ | | Li₂Si₂O₅ | Li₂Si₂O₅ | | Li₂Si₂O₅ | | Li₂Si₂O₅ | | |
| | Li₂SiO₃ | Li₂SiO₃ | Li₃PO₄ | Li₂Si₂O₅ | Li₃PO₄ | Li₃PO₄ | Li₂Si₂O₅ | Li₃PO₄ | Li₂Si₂O₅ | Li₃PO₄ | Li₂Si₂O₅ | |
| | Cristobalit | Li₃PO₄ | WP₂O₇ | Li₃PO₄ | Li₂SiO₃ | Li₂SiO₃ | Li₃PO₄ | Li₂SiO₃ | Li₂SiO₃ | Li₂SiO₃ | Li₃PO₄ | |
| | | Cristobalit | Li₂WO₄ | | Cristobalit | Cristobalit | Li₂SiO₃ | Cristobalit | | LiSr (PO₄) | | |
| **Farbeindruck der Glaskeramik** | gelb | gelb | orangebraun | gelb | gelbbraun | gelb | gelb | gelb | gelb | gelb | gelb | gelb |

**Tabelle 14**

| **Beispiel** | **66** | **67** | **68** | **69** | **70** | **71** | **72** | **73** | **74** | **76** | **77** | **78** |
|---|---|---|---|---|---|---|---|---|---|---|---|---|
| T_{g} /°C | | 464,4 | 475 | 474,6 | | 463 | 459 | 456 | 465 | | 471,2 | 462,7 |
| T_{S1} /°C | 1400 | 1400 | 1500 | 1500 | 1450 | 1450 | 1450 | 1550 | 1500 | 1450 | 1450 | 1450 |
| t_{S1} /min | 60 | 60 | 30 | 30 | 120 | 120 | 120 | 120 | 120 | 300 | 120 | 120 |
| T_{S2} /°C | | | 1650 | 1650 | | | | | | | | |
| t_{S2} /min | | | 30 | 30 | | | | | | | | |
| T_{N} /°C | 490 | 490 | 490 | 490 | 470 | 480 | 480 | 470 | 480 | 490 | 490 | 490 |
| t_{N} /min | 10 | 10 | 10 | 10 | 10 | 10 | 10 | 10 | 0 | 60 | 60 | 10 |
| **1 .Wärmebehandlung** | | | | | | | | | | | | |
| Temperatur /°C | 900 | 900 | 800 | 800 | 640 | 840 | 840 | 710 | 750 | 750 | 750 | 850 |
| Dauer /min | 60 | 60 | 30 | 30 | 30 | 15 | 30 | 30 | 60 | 30 | 30 | 30 |
| **Bestrahlung Glaskeramik** | | | | | | | | | | | | |
| Strahlungsquelle | LED | LED | LED | LED | LED | LED | LED | LED | LED | LED | LED | LED |
| Wellenlänge /nm | 310 | 310 | 310 | 310 | 310 | 310 | 310 | 310 | 310 | 310 | 310 | 310 |
| Dauer /min | 15 | 15 | 15 | 15 | 15 | 20 | 60 | 5 | 30 | 30 | 30 | 15 |
| Intensität /mA | 400 | 400 | 400 | 400 | 400 | 400 | 400 | 400 | 400 | 400 | 400 | 400 |
| **2 .Wärmebehandlung** | | | | | | | | | | | | |
| Temperatur /°C | 550 | 550 | 550 | 550 | 640 | 650 | 650 | 710 | 750 | 600 | 550 | 850 |
| Dauer /min | 15 | 15 | 60 | 15 | 5 | 30 | 15 | 30 | 5 | 15 | 60 | 60 |
| **Kristallphasen** | | | | Li₂Si₂O₅ | Li₂SiO₃ | | Li₂Si₂O₅ | Li₂Si₂O₅ | Li₂Si₂O₅ | Li₂Si₂O₅ | Li₂Si₂O₅ | Li₂Si₂O₅ |
| | Li₂Si₂O₅ | Li₂Si₂O₅ | Li₂Si₂O₅ | α-Quarz | Li₃PO₄ | | Li₃PO₄ | Li₃PO₄ | Li₃PO₄ | Li₃PO₄ | Li₃PO₄ | Li₃PO₄ |
| | Li₃PO₄ | Li₃PO₄ | Li₃PO₄ | Cristo-balit | | | | Cristo-balit | α-Quarz | α-Quarz | α-Quarz | Li₂SiO₃ |
| σ_{B} /MPa | | | | | 314 | | 327 | | | | 207 | |
| CR (Transluzenz) | | | | | 75,59 | 84,61 | | | 66,09 | 64,24 | 64,5 | |
| L* | | | | | 91,8 | 86,43 | | | 91,52 | 90,89 | 86,71 | |
| a* | | | | | -2,8 | 2,6 | | | 0,89 | -2,09 | 1,44 | |
| b* | | | | | 20,65 | 25,06 | | | 17,03 | 12,76 | 16,92 | |
| **Farbeindruck der Glaskeramik** | braun | braun | gelbleicht braun | leicht gelb | gelb | gelb | gelb | gelbgrau | gelb | gelb | gelb | gelb |

**Tabelle 15**

| **Beispiel** | **80** | **81** | **82** | **83** | **84** | **85** | **86** | **87** | **88** | **89** | **90** |
|---|---|---|---|---|---|---|---|---|---|---|---|
| T_{g} /°C | 476,7 | | | 471,2 | 476,6 | 469 | 467,9 | 467,3 | 475,6 | 486,8 | 480,6 |
| T_{S1} /°C | 1500 | 1500 | 1500 | 1500 | 1500 | 1500 | 1500 | 1500 | 1500 | 1500 | 1500 |
| t_{S1} /min | 30 | 30 | 15 | 30 | 30 | 30 | 30 | 30 | 30 | 30 | 30 |
| T_{S2} /°C | 1650 | 1650 | 1650 | 1650 | 1650 | 1650 | 1650 | 1650 | 1650 | 1650 | 1650 |
| t_{S2} /min | 30 | 30 | 45 | 30 | 30 | 30 | 30 | 30 | 30 | 30 | 30 |
| T_{N} /°C | 500 | 490 | 490 | 500 | 520 | 490 | 490 | 490 | 500 | 490 | 500 |
| t_{N} /min | 10 | 0 | 0 | 10 | 10 | 10 | 10 | 10 | 10 | 10 | 10 |
| **1 .Wärmebehandlung** | | | | | | | | | | | |
| Temperatur /°C | 830 | 750 | 750 | 800 | 780 | 730 | 800 | 750 | 800 | 830 | 800 |
| Dauer /min | 60 | 30 | 30 | 60 | 60 | 60 | 60 | 10 | 60 | 60 | 60 |
| **Bestrahlung Glaskeramik** | | | | | | | | | | | |
| Strahlungsquelle | LED | LED | LED | LED | LED | LED | LED | LED | LED | LED | LED |
| Wellenlänge /nm | 310 | 310 | 310 | 310 | 310 | 310 | 310 | 310 | 310 | 310 | 310 |
| Dauer /min | 15 | 15 | 15 | 15 | 15 | 15 | 15 | 15 | 15 | 15 | 15 |
| Intensität /mA | 400 | 400 | 400 | 400 | 400 | 400 | 400 | 400 | 400 | 400 | 400 |
| **2 .Wärmebehandlung** | | | | | | | | | | | |
| Temperatur /°C | 550 | 550 | 550 | 550 | 550 | 550 | 550 | 550 | 550 | 550 | 550 |
| Dauer /min | 10 | 15 | 15 | 15 | 10 | 15 | 15+45 | 10 | 10 | 15 | 15 |
| **Kristallphasen** | | | | | | α-Quarz | | α-Quarz | | | α-Quarz |
| | α-Quarz | α-Quarz | α-Quarz | α-Quarz | α-Quarz | Li₂Si₂O₅ | α-Quarz | Li₂Si₂O₅ | Li_{0,155}Al_{0,155}-Si_{0,845}O₂ | α-Quarz | Li₂Si₂O₅ |
| | Li₂Si₂O₅ | Li₂Si₂O₅ | Li₂Si₂O₅ | Li₂Si₂O₅ | Li₂Si₂O₅ | Li₃PO₄ | Li₂Si₂O₅ | Li₃PO₄ | Li₂Si₂O₅ | Li₂Si₂O₅ | Li₃PO₄ |
| | Li₃PO₄ | Li₃PO₄ | Li₃PO₄ | Li₃PO₄ | Li₃PO₄ | Ca₂Al₂SiO₇ | Li₃PO₄ | Cristobalit | Li₃PO₄ | Li₃PO₄ | Cristobalit |
| **Farbeindruck der Glaskeramik** | gelb | gelb | gelb | gelb | gelb | gelb | gelb | leicht gelb | gelb | gelb | leicht gelb |

### Beispiel 91: Farbänderung durch Bestrahlung mit Röntgenstrahlung und Wärmebehandlung

Es wurde eine Glaskeramik entsprechend Beispiel 74 hergestellt, mit dem Unterschied, dass die Keimbildungsdauer t_{N} 10 min betrug. Nach der Wärmebehandlung zur Kristallisation wurden Li₂Si₂O₅ als Hauptkristallphase sowie Li₃PO₄ und Tiefquarz als Nebenkristallphasen bestimmt. Die Glaskeramik wurde für 2 Stunden mit Cu-K_{α}-Strahlung unter Verwendung eines Röntgendiffraktometers (D8 Advance, Bruker, Karlsruhe, Germany) bei einer Betriebsspannung von 40 kV bestrahlt. Nach der Bestrahlung mit der hochenergetischen Strahlung, die bereits mit einer Wärmewirkung einhergeht, wurde bereits eine leichte Gelbfärbung der Glaskeramik beobachtet. Durch die anschließende Wärmebehandlung (750°C, 10 min) wurde die Gelbfärbung verstärkt.

### Vergleichsbeispiele 75 und 79: Keine Bestrahlung des Glases oder der Glaskeramik

Die Lithiumsilikatgläser der Vergleichsbeispiele 75 und 79 wurden einer Wärmebehandlung zur Keimbildung und einer Wärmebehandlung zur Kristallisation unterzogen. Im Gegensatz zu den Gläsern und Glaskeramiken, die zusätzlich einer Bestrahlung und Wärmebehandlung ausgesetzt wurden, war z.B. die in Vergleichsbeispiel 75 hergestellte Glaskeramik ungefärbt.

**Tabelle 16**

| **Beispiel** | **75** | **79** |
|---|---|---|
| T_{g} /°C | 465 | 462,7 |
| T_{S1} /°C | 1500 | 1450 |
| t_{S1} /min | 120 | 120 |
| T_{N} /°C | 480 | 490 |
| t_{N} /min | 10 | 10 |
| **1.Wärmebehandlung** | | |
| Temperatur /°C | 750 | 840 |
| Dauer /min | 30 | 30 |
| **Kristallphasen** | Li₂Si₂O₅ | Li₂Si₂O₅ |
| | Li₃PO₄ | Li₃PO₄ |
| | α-Quarz | Li₂SiO₃ |
| σ_{B} /MPa | 331 | |
| **Farbeindruck der Glaskeramik** | ungefärbt | ungefärbt |

Die Glaskeramik gemäß Vergleichsbeispiel 79 wies genau wie die Glaskeramik gemäß Beispiel 74 eine Bruchzähigkeit von 2,46 MPa m^{0,5} (bestimmt als K_{IC}-Wert nach der SEVNB-Methode, beschrieben in ISO 6872 von 2008) auf.

## Patentansprüche

1. Verfahren zur Herstellung einer mehrfarbigen Dentalrestauration, bei dem
a) einem Glas oder einer Glaskeramik die Form einer Dentalrestauration gegeben wird, und
b) in mindestens einem Teil des Glases oder der Glaskeramik eine Farbänderung bewirkt wird, indem dieser Teil mit künstlicher elektromagnetischer Strahlung bestrahlt und dieser bestrahlte Teil einer Wärmebehandlung unterzogen wird.

2. Verfahren nach Anspruch 1, bei dem das Glas und die Glaskeramik mindestens eine oxidierbare Komponente und mindestens eine reduzierbare Färbekomponente enthalten.

3. Verfahren nach Anspruch 1 oder 2, bei dem das Glas und die Glaskeramik Ce, berechnet als CeO₂, bevorzugt in einer Menge von 0,01 bis 1,5, insbesondere 0,03 bis 1 Gew.-%, enthalten.

4. Verfahren nach einem der Ansprüche 1 bis 3, bei dem das Glas und die Glaskeramik Ag, berechnet als Ag₂O, bevorzugt in einer Menge von 0,0005 bis 1,3, insbesondere 0,002 bis 0,7 Gew.-%, enthalten.

5. Verfahren nach einem der Ansprüche 1 bis 4, bei dem das Glas und die Glaskeramik Au, berechnet als Au₂O, bevorzugt in einer Menge von 0,0001 bis 0,65, insbesondere 0,0003 bis 0,25, besonders bevorzugt 0,003 bis 0,2 Gew.-%, enthalten.

6. Verfahren nach einem der Ansprüche 1 bis 5, bei dem das Glas und die Glaskeramik Ce sowie Ag und/oder Au enthalten.

7. Verfahren nach einem der Ansprüche 1 bis 6, bei dem das Glas und die Glaskeramik Cl, Br und/oder I, insbesondere Cl, in einer Menge von 0,0001 bis 0,9, besonders bevorzugt 0,0005 bis 0,7 Gew.-%, enthalten.

8. Verfahren nach einem der Ansprüche 1 bis 7, bei dem das Glas und die Glaskeramik P₂O₅, insbesondere in einer Menge von 0,5 bis 11,0, besonders bevorzugt 0,9 bis 8,0 Gew.-%, enthalten.

9. Verfahren nach einem der Ansprüche 1 bis 8, bei dem das Glas und die Glaskeramik mindestens eine und bevorzugt alle der folgenden Komponenten in den angegebenen Mengen enthalten:
| Komponente | Gew.-% |
|---|---|
| SiO₂ | 61,0 - 88,0 |
| Li₂O | 5,0 - 24,0 |
| Al₂O₃ | 0 - 14,0 |
| P₂O₅ | 0,5 - 11,0 |
| Ce, berechnet als CeO₂ | 0,01 - 1,5 |
| Ag, berechnet als Ag₂O | 0,0005 - 1,3 |
| Au, berechnet als Au₂O | 0,0001 - 0,65 |

10. Verfahren nach einem der Ansprüche 1 bis 9, bei dem die Strahlung eine Wellenlänge von höchstens 380 nm, insbesondere im Bereich von 100 bis 360 nm, besonders bevorzugt im Bereich von 250 bis 350 nm, am meisten bevorzugt im Bereich von 300 bis 310 nm, hat.

11. Verfahren nach einem der Ansprüche 1 bis 10, bei dem die Wärmebehandlung bei einer Temperatur im Bereich von 300 bis 1000°C, insbesondere 400 bis 950°C, besonders bevorzugt 450 bis 850°C, erfolgt.

12. Verfahren nach einem der Ansprüche 1 bis 11, bei dem die Wärmebehandlung für eine Dauer von bis zu 120 min, insbesondere bis zu 60 min, erfolgt.

13. Verfahren nach einem der Ansprüche 1 bis 12, bei dem das Glas und die Glaskeramik ausgewählt sind aus der Gruppe bestehend aus Lithiumsilikatglas, Lithiumalumosilikatglas, Lithiumsilikat-Glaskeramik, Lithiumalumosilikat-Glaskeramik und Quarz-Glaskeramik.

14. Verfahren nach Anspruch 13, bei dem die Glaskeramik Lithiummetasilikat, Lithiumdisilikat, Tiefquarz oder Lithiumalumosilikat als Hauptkristallphase enthält.

15. Verfahren nach einem der Ansprüche 1 bis 14, bei dem dem Glas und der Glaskeramik durch Verpressen oder maschinelle Bearbeitung die Form der Dentalrestauration gegeben werden.

16. Verfahren nach Anspruch 15, bei dem die maschinelle Bearbeitung in einem CAD/CAM-Verfahren erfolgt.

17. Verfahren nach einem der Ansprüche 1 bis 16, bei dem die Dentalrestauration eine Brücke, ein Inlay, ein Onlay, ein Veneer, ein Abutment, eine Teilkrone, eine Krone oder eine Schale ist.

18. Mehrfarbige Dentalrestauration, die gemäß einem Verfahren nach einem der Ansprüche 1 bis 17 erhältlich ist.

19. Verwendung eines Glases oder einer Glaskeramik als Dentalmaterial, insbesondere zur Herstellung einer mehrfarbigen Dentalrestauration, wobei in mindestens einem Teil des Glases oder der Glaskeramik eine Farbänderung bewirkt wird, indem dieser Teil mit künstlicher elektromagnetischer Strahlung bestrahlt und dieser bestrahlte Teil einer Wärmebehandlung unterzogen wird.

20. Verwendung nach Anspruch 19, bei der das Glas und die Glaskeramik einem Verfahren nach einem der Ansprüche 1 bis 17 unterzogen werden.
